# EUROPÄISCHE PATENTSCHRIFT

(11) **EP 3 075 330 B1**
(45) Veröffentlichungstag und Bekanntmachung des Hinweises auf die Patenterteilung: **12.06.2019**
(21) Anmeldenummer: 16163546.1
(22) Anmeldetag: 01.04.2016
(51) Int. Cl.: A61B 17/15

(54) **MEDIZINISCHE FÜHRUNGSVORRICHTUNG**
MEDICAL GUIDING DEVICE
DISPOSITIF DE GUIDAGE CHIRURGICAL

(30) Priorität: 02.04.2015 US 201562142061 P
(43) Veröffentlichungstag der Anmeldung: 05.10.2016
(73) Patentinhaber: Aesculap AG, 78532 Tuttlingen (DE)
(72) Erfinder: Firmbach, Franz-Peter, 78576 Emmingen-Liptingen (DE); Mihalko, William, Germantown, TN 38138 (US); Nonnenmann, Martin, 78573 Wurmlingen (DE)
(74) Vertreter: Hoeger, Stellrecht & Partner Patentanwälte mbB

(56) Entgegenhaltungen:
- EP-A1- 1 442 712
- EP-A2- 1 779 790
- WO-A1-2005/048851
- WO-A2-2010/128409
- DE-A1- 19 516 294

## Beschreibung

Die vorliegende Erfindung betrifft eine medizinische Führungsvorrichtung zum Bearbeiten eines Knochens, welche Führungsvorrichtung eine Halteeinrichtung zum Festlegen der Führungsvorrichtung am Knochen und eine Sägeschablone mit einem Führungsschlitz für ein Sägeblatt umfasst, wobei die Halteeinrichtung und die Sägeschablone in einer Justierstellung miteinander in Eingriff stehen und relativ zueinander verstellbar angeordnet sind, wobei die Halteeinrichtung und die Sägeschablone von der Justierstellung in eine Sägestellung bringbar sind, in welcher sie relativ zueinander unbeweglich festgelegt sind, welche Führungsvorrichtung eine Koppeleinrichtung umfasst zum Koppeln der Halteeinrichtung und der Sägeschablone in der Justierstellung und in der Sägestellung, wobei die Koppeleinrichtung erste und zweite Koppelelemente umfasst, die einerseits an der Halteeinrichtung und andererseits an der Sägeschablone angeordnet oder ausgebildet sind und in der Justierstellung miteinander in Eingriff stehen und in der Sägestellung kraft- und/oder formschlüssig aneinander gehalten sind, wobei das erste Koppelelement in Form einer Gelenkkugel ausgebildet ist, wobei das zweite Koppelelement in Form einer Gelenkkugelaufnahme ausgebildet ist, wobei die Gelenkkugelaufnahme eine hohlzylindrische oder im Wesentlichen hohlzylindrische Innenkontur definiert und es ermöglicht, die darin gehaltene Gelenkkugel (96) nicht nur um einen von der Gelenkkugel (96) definierten Mittelpunkt zu rotieren, sondern die Gelenkkugel (96) relativ zur Gelenkkugelaufnahme (98) auch parallel zu einer von der Gelenkkugelaufnahme (98) definierten Längsachse (104) zu verschieben.

Medizinische Führungsvorrichtungen der eingangs beschriebenen Art werden insbesondere in der Chirurgie genutzt, um Knochenflächen zu präparieren, an welche Anlageflächen von Endoprothesen angelegt werden. Die Halteeinrichtung wird dabei üblicherweise zunächst am Knochen festgelegt, der präpariert werden soll. Beispielsweise wird zur Vorbereitung einer Implantation einer Kniegelenkendoprothese die Halteeinrichtung an einer Tibia oder an einem Femur festgelegt. In einem nächsten Schritt kann eine zum Beispiel mit der Halteeinrichtung gekoppelte Sägeschablone relativ zu dieser ausgerichtet werden. Danach können mit Hilfe der Sägeschablone ein oder mehrere Schnitte am Knochen vorgenommen werden, beispielsweise mit einer oszillierenden Säge, deren Sägeblatt durch die Sägeschablone geführt wird.

Ein Problem bei medizinischen Führungsvorrichtungen der eingangs beschriebenen Art ist es insbesondere, diese einfach und schnell am Knochen zu befestigen sowie die Sägeschablone in gewünschter Weise auszurichten.

Ein Befestigungssystem für Referenzierungseinheiten für die Computer-unterstützte Chirurgie ist aus der WO 2010/128409 A2 bekannt. Eine Halterung zum Festlegen von Instrumentenführungen ist in der EP 1 779 790 A2 offenbart. In der EP 1 442 712 A1 ist ein universales Ausrichtinstrument beschrieben. Eine multidirektionale Osteotomielehre für Knochenumstellungsoperationen beim Menschen ist aus der DE 195 16 294 A1 bekannt. Ferner ist in der WP 2005/048851 A1 ein einstellbares chirurgisches Schneidesystem offenbart.

Es ist daher eine Aufgabe der vorliegenden Erfindung, die Handhabung medizinischer Führungsvorrichtungen der eingangs beschriebenen Art zu verbessern.

Diese Aufgabe wird bei einer medizinischen Führungsvorrichtung der eingangs beschriebenen Art erfindungsgemäß dadurch gelöst, dass es die Gelenkkugelaufnahme ermöglicht, die darin gehaltene Gelenkkugel nicht nur um einen von der Gelenkkugel definierten Mittelpunkt zu rotieren, sondern die Gelenkkugel relativ zur Gelenkkugelaufnahme auch parallel zu einer von der Gelenkkugelaufnahme definierten Längsachse zu verschieben, und dass es die Koppeleinrichtung ermöglicht, nach gewünschter Ausrichtung der Sägeschablone die ersten und zweiten Koppelelemente von der Justierstellung in die Sägestellung zu überführen, um in dieser Sägestellung die Sägeschablone in der definierten Ausrichtung zu halten.

Eine medizinische Führungsvorrichtung mit einer solchen Koppeleinrichtung ermöglicht es, die Sägeschablone relativ zur Halteeinrichtung und, wenn die Halteeinrichtung am Knochen festgelegt ist, relativ zum Knochen in definierter Weise in der Justierstellung auszurichten. Ist die Sägeschablone wie gewünscht ausgerichtet, ermöglicht es die Koppeleinrichtung die ersten und zweiten Koppelelemente von der Justierstellung in die Sägestellung zu überführen, um in dieser die Sägeschablone in der definierten Ausrichtung zu halten, so dass ein Sägeschnitt zur Bearbeitung des Knochens hochpräzise ausgeführt werden kann. Die vorgeschlagene Führungsvorrichtung ermöglicht es also insbesondere, zunächst die Halteeinrichtung, optional auch mit daran beweglich gehaltener Sägeschablone, grob am Knochen zu positionieren und erst dann eine Feinausrichtung der Sägeschablone vorzunehmen. Günstig ist es, dass das erste Koppelelement in Form einer Gelenkkugel ausgebildet ist und dass das zweite Koppelelement in Form einer Gelenkkugelaufnahme ausgebildet ist. Die Gelenkkugel und die Gelenkkugelaufnahme sind vorzugsweise derart bemessen, dass eine Rotation zumindest in der Justierstellung relativ zueinander um einen gemeinsamen Drehpunkt möglich ist. Vorteilhafterweise definiert die Gelenkkugelaufnahme eine hohlzylindrische oder im Wesentlichen hohlzylindrische Innenkontur. Eine solche Gelenkkugelaufnahme ermöglicht es insbesondere, eine darin gehaltene Gelenkkugel nicht nur um einen von der Gelenkkugel definierten Mittelpunkt zu rotieren, sondern die Gelenkkugel relativ zur Gelenkkugelaufnahme auch parallel zu einer von der Gelenkkugelaufnahme definierten Längsachse zu verschieben. So können auf einfache Weise mehrere Freiheitsgrade vorgegeben werden, die eine definierte und einfache Ausrichtung der Sägeschablone relativ zur Halteeinrichtung ermöglichen.

Um eine Position und/oder eine Orientierung der Sägeschablone hochpräzise vornehmen zu können, ist es günstig, wenn an der Halteeinrichtung und/oder an der Sägeschablone eine Schnittstelle für eine medizinische Referenzierungseinrichtung angeordnet oder ausgebildet ist, deren Position und/oder Orientierung im Raum durch ein medizinisches Navigationssystem detektierbar ist und/oder wenn an der Halteeinrichtung und/oder an der Sägeschablone eine medizinische Referenzierungseinrichtung angeordnet oder ausgebildet ist, deren Position und/oder Orientierung im Raum durch ein medizinisches Navigationssystem detektierbar ist. Die vorgeschlagene Weiterbildung ermöglicht es insbesondere, eine Position und/oder eine Orientierung der Halteeinrichtung und/oder der Sägeschablone mittels eines Navigationssystems hochpräzise zu bestimmen. Ist insbesondere eine Positionierung und/oder eine Orientierung der Halteeinrichtung am zu bearbeitenden Knochen bekannt, so kann die Sägeschablone entsprechend relativ zur Halteeinrichtung wie gewünscht ausgerichtet werden, so dass Knochenflächen exakt vorbereitet werden können, um daran Implantatteile einer zu implantierenden Prothese temporär oder dauerhaft festzulegen.

Vorteilhaft ist es, wenn die Schnittstelle in Form eines ersten Verbindungselements ausgebildet ist, welches korrespondierend zu einem zweiten Verbindungselement der medizinischen Referenzierungseinrichtung ausgebildet ist und wenn das erste und das zweite Verbindungselement in einer Verbindungsstellung in Eingriff stehen und in einer Reinigungsstellung außer Eingriff stehen. Eine solche Schnittstelle ermöglicht es, die Referenzierungseinrichtung insbesondere vollständig von der Halteeinrichtung und/oder von der Sägeschablone zu trennen. Dies hat Vorteile bei der Reinigung aller Komponenten nach einem chirurgischen Eingriff. Ferner bietet es auch die Option, die Referenzierungseinrichtung von der Halteeinrichtung und/oder von der Sägeschablone zu entfernen vor Ausführen eines Sägeschnitts. Die Referenzierungseinrichtung ist nach dem Entfernen insbesondere bei eingeschränkten räumlichen Verhältnissen im Bereich des Operationssitus für einen Operateur nicht mehr im Weg.

Auf einfache Weise lässt sich eine Referenzierungseinrichtung mit der Halteeinrichtung und/oder der Sägeschablone verbinden, wenn das erste Verbindungselement in Form eines Verbindungsvorsprungs oder einer Verbindungsaufnahme ausgebildet ist. Ein korrespondierendes Verbindungselement kann dann insbesondere an der Referenzierungseinrichtung ausgebildet sein, um vorzugsweise in einer eindeutigen Ausrichtung relativ zueinander die Referenzierungseinrichtung mit der Halteeinrichtung und/oder der Sägeschablone zu verbinden.

Günstigerweise trägt die medizinische Referenzierungseinrichtung mindestens ein Markerelement, dessen Position im Raum durch ein medizinisches Navigationssystem detektierbar ist. Vorzugsweise umfasst die medizinische Referenzierungseinrichtung zwei, drei, vier oder noch mehr Markerelemente. Je größer deren Zahl, umso genauer können eine Position und/oder eine Orientierung der Referenzierungseinrichtung im Raum durch das Navigationssystem detektiert werden.

Gemäß einer weiteren bevorzugten Ausführungsform der Erfindung kann vorgesehen sein, dass die Koppeleinrichtung derart ausgebildet ist, dass die Sägeschablone und die Halteeinrichtung in der Justierstellung relativ zueinander verschiebbar aneinander gelagert sind. Diese Ausgestaltung definiert somit mindestens einen Translationsfreiheitsgrad, um die Sägeschablone relativ zur Halteeinrichtung in mindestens einer Richtung zu bewegen.

Ferner ist es vorteilhaft, wenn das zweite Koppelelement eine Koppelelementlängsachse definiert und wenn die Sägeschablone und die Halteeinrichtung in der Justierstellung relativ zueinander parallel zur Koppelelementlängsachse verschiebbar aneinander gelagert sind. Auf diese Weise kann durch entsprechende Ausgestaltung eine definierte Führung für eine Verschiebebewegung vorgegeben werden, und zwar insbesondere parallel zur Koppelelementlängsachse.

Günstig ist es, wenn die Koppeleinrichtung derart ausgebildet ist, dass die Sägeschablone und die Halteeinrichtung in der Justierstellung relativ zueinander um eine Schwenkachse verschwenkbar aneinander gelagert sind. Diese Ausgestaltung ermöglicht es insbesondere, eine Neigung des Führungsschlitzes in gewünschter Weise vorzugeben, beispielsweise relativ zum Knochen, an welchem die Führungsvorrichtung festgelegt ist, oder bezogen auf die Halteeinrichtung. Selbstverständlich kann die Koppeleinrichtung auch zwei oder drei oder mehr Schwenkachsen definieren, die nicht parallel zueinander verlaufen. Dies ermöglicht es insbesondere, die Sägeschablone relativ zur Halteeinrichtung um mehrere Freiheitsgrade in gewünschter Weise auszurichten.

Besonders vorteilhaft ist es, wenn die Koppeleinrichtung derart ausgebildet ist, dass die Sägeschablone und die Halteeinrichtung in der Justierstellung relativ zueinander um einen Drehpunkt rotierbar aneinander gelagert sind. Diese Ausgestaltung kann insbesondere auch in Kombination mit einer verschiebbaren Lagerung der ersten und zweiten Koppelelemente relativ zueinander vorgesehen sein. Eine rotierbare Lagerung um einen Drehpunkt ermöglicht insbesondere eine einfache und präzise Ausrichtung der Sägeschablone relativ zur Halteeinrichtung.

Auf besonders einfache Weise ausbilden lässt sich die Koppeleinrichtung, wenn sie ein Kugelgelenk umfasst.

Um einen Aufbau der Führungseinrichtung möglichst einfach zu gestalten, ist es günstig, wenn die Gelenkkugelaufnahme mindestens zwei hohlzylindrische Wandabschnitte umfasst, welche einen Innendurchmesser definieren, welcher einem Außendurchmesser der Gelenkkugel entspricht oder im Wesentlichen entspricht. Die Abmessungen sind vorzugsweise so gewählt, dass in der Justierstellung eine Bewegung der Gelenkkugel und der Gelenkkugelaufnahme relativ zueinander möglich ist.

Ein besonders kompakter Aufbau der Halteeinrichtung lässt sich insbesondere dadurch erreichen, dass sie einen Halteeinrichtungsgrundkörper aufweist und dass das erste Koppelelement am Halteeinrichtungsgrundkörper gehalten ist. Beispielsweise ermöglicht es dies, den Halteeinrichtungsgrundkörper an einem Knochen festzulegen und das zweite Koppelelement der Sägeschablone mit dem ersten Koppelelement in Eingriff zu bringen.

Gemäß einer weiteren bevorzugten Ausführungsform der Erfindung kann vorgesehen sein, dass zwischen dem ersten Koppelelement und dem Halteeinrichtungsgrundkörper eine Einschnürung oder ein Verbindungssteg ausgebildet ist und dass die Einschnürung oder der Verbindungssteg in der Justier- und in der Koppelstellung einen Koppelspalt der Gelenkkugelaufnahme durchsetzt. Durch diese besondere Ausgestaltung kann insbesondere eine Gelenkkugel mit einer der oben beschriebenen vorteilhaften Gelenkkugelaufnahmen in definierter Weise in Eingriff gebracht werden. Ferner kann zudem eine grobe Führung zwischen den beiden Koppelelementen ausgebildet werden, wenn der Koppelspalt im Wesentlichen den äußeren Abmessungen der Einschnürung entspricht, so dass die Einschnürung nur parallel zu einer Längsachse des zweiten Koppelelements relativ zu diesem bewegbar ist.

Ferner kann es vorteilhaft sein, wenn die Führungseinrichtung eine Feststelleinrichtung zum kraft- und/oder formschlüssigen Halten der Halteeinrichtung und der Sägeschablone in der Sägestellung umfasst. Die Feststelleinrichtung ermöglicht es insbesondere auf einfache Weise, die Führungsvorrichtung von der Justierstellung in die Sägestellung zu überführen und/oder umgekehrt.

Auf besonders einfache Weise lassen sich die Sägeschablone und die Halteeinrichtung in der Sägestellung aneinander festlegen, wenn die Feststelleinrichtung in Form einer Rast-, Schnapp- und/oder Klemmeinrichtung ausgebildet ist.

Besonders einfach und kompakt ausbilden lässt sich die Feststelleinrichtung, wenn sie mindestens ein erstes Feststellelement umfasst, welches in der Sägestellung direkt oder indirekt das erste Koppelelement gegen das zweite Koppelelement drückt. Insbesondere kann so einfach und sicher eine Klemmung der Koppelelemente aneinander erreicht werden.

Vorteilhaft ist es, wenn die Feststelleinrichtung mindestens ein zweites Feststellelement umfasst und wenn das mindestens eine erste Feststellelement das mindestens eine zweite Feststellelement in der Sägestellung gegen das erste Koppelelement und dieses gegen das zweite Koppelelement drückt. Auf diese Weise kann eine Andrückkraft vom ersten Feststellelement über das zweite Feststellelement indirekt auf das erste und/oder zweite Koppelelement übertragen werden.

Um bereits eine Haltekraft zum Halten der Halteeinrichtung und der Sägeschablone aneinander in der Justierstellung zu ermöglichen, ist es günstig, wenn das mindestens eine zweite Feststellelement ein an der Sägeschablone angeordnetes oder ausgebildetes Blattfederelement umfasst. Das Blattfederelement, denkbar sind auch andere Arten von Federelementen, ermöglicht es insbesondere bereits in der Justierstellung, eine definierte Andrück- oder Klemmkraft auszuüben, um die Halteeinrichtung und die Sägeschablone in definierter Weise aneinander zu halten.

Vorzugsweise umfasst das mindestens eine zweite Feststellelement einen Andrückkörper. Dieser kann insbesondere aus einem entsprechend geeigneten Material gebildet oder hinreichend unverformbar ausgebildet sein, dass eine definierte Klemmkraft mit der Feststelleinrichtung ausgeübt werden kann, um die Halteeinrichtung und die Sägeschablone in der Justierstellung und/oder in der Sägestellung aneinander zu halten.

Vorteilhaft ist es, wenn das Blattfederelement an einem freien Ende oder im Bereich eines freien Endes den Andrückkörper trägt. Diese Ausgestaltung gestattet es insbesondere, mittels des Blattfederelements den Andrückkörper in definierter Weise direkt oder indirekt gegen das erste und/oder das zweite Koppelelement zu drücken.

Um einen besonders kompakten Aufbau der Feststelleinrichtung zu ermöglichen, ist es günstig, wenn der Andrückkörper eine Andrückkörperlängsachse definiert, die parallel oder im Wesentlichen parallel zur Koppelelementlängsachse verläuft. Beispielsweise kann der Andrückkörper mit seiner Andrückkörperlängsachse im Koppelspalt parallel zu diesem ausgerichtet angeordnet sein.

Für eine besonders einfache und intuitive Handhabung der Führungseinrichtung ist es vorteilhaft, wenn das mindestens eine zweite Feststellelement beweglich an der Sägeschablone angeordnet oder ausgebildet ist. Ist beispielsweise die Halteeinrichtung bereits am Knochen festgelegt, muss ein Operateur lediglich noch die Sägeschablone halten und kann durch Bewegen des mindestens einen zweiten Feststellelements die Sägeschablone und die Halteeinrichtung von der Justierstellung in die Sägestellung überführen.

Ein besonders kompakter Aufbau der Führungsvorrichtung kann insbesondere dadurch erreicht werden, dass das mindestens eine zweite Feststellelement in einem Spalt oder einer Aussparung zwischen den mindestens zwei hohlzylindrischen Wandabschnitten gehalten ist. Insbesondere kann es sich bei dem Spalt um einen oder den oben beschriebenen Koppelspalt handeln. Beispielsweise können ein, zwei oder mehr Andrückkörper zwischen den zwei hohlzylindrischen Wandabschnitten angeordnet sein.

Für eine einfache und intuitive Betätigung der Führungsvorrichtung ist es günstig, wenn das mindestens eine erste Feststelleelement einen um eine Exzenterschwenkachse verschwenkbaren Exzenterkörper umfasst. Durch einfaches Verschwenken des Exzenterkörpers um die Exzenterschwenkachse kann so die Führungsvorrichtung von der Justierstellung in die Sägestellung überführt werden und/oder umgekehrt. Einen Exzenterkörper in der beschriebenen Weise vorzusehen ermöglicht es insbesondere, eine Klemmkraft zwischen den zusammenwirkenden Koppelelementen der Kopplungseinrichtung stufenlos zu ändern. Außerdem können mit einer solchen Koppeleinrichtung alle Freiheitsgrade der Kopplungseinrichtung insbesondere durch eine einfache Verschwenkbewegung des Exzenterkörpers blockiert werden. Es sind also nicht verschiedene Justierschrauben oder dergleichen erforderlich, um die Sägeschablone und die Halteeinrichtung in einer gewünschten Weise relativ zueinander auszurichten, sondern lediglich eine einfache Verschwenkung des Exzenterkörpers um die Exzenterschwenkachse.

Um eine optimale Klemmung der Koppelelemente der Koppeleinrichtung aneinander zu erreichen, ist es vorteilhaft, wenn die Exzenterachse quer, insbesondere senkrecht, zur Koppelelementlängsachse verläuft. Insbesondere kann die Exzenterachse senkrecht zur Koppelelementlängsachse verlaufen.

Ferner kann es günstig sein, wenn die Exzenterachse parallel oder im Wesentlichen parallel zum Führungsschlitz verläuft. Insbesondere kann das mindestens eine erste Feststellelement derart angeordnet oder ausgebildet sein, dass in der Justierstellung der Führungsschlitz vom mindestens einen ersten Feststellelement verdeckt ist und in der Sägestellung das mindestens eine erste Feststellelement den Führungsschlitz der Sägeschablone freigibt. So kann insbesondere sichergestellt werden, dass nur dann ein Sägeschnitt ausgeführt wird, wenn die Sägeschablone sicher an der Halteeinrichtung in der Sägestellung gehalten ist.

Um eine einfache Handhabung der Feststelleinrichtung zu ermöglichen, ist es günstig, wenn das mindestens eine erste Feststellelement ein Betätigungsglied umfasst. Vorzugsweise ist das Betätigungsglied insbesondere vom Exzenterkörper abstehend angeordnet oder ausgebildet. Beispielsweise kann es laschen- oder lappenförmig ausgebildet sein, so dass ein Operateur den Exzenterkörper auf einfache Weise verschwenken kann, um die Führungsvorrichtung von der Justierstellung in die Sägestellung zu überführen und/oder umgekehrt.

Um die Führungsvorrichtung in definierter Weise an einem Knochen festlegen zu können, ist es vorteilhaft, wenn an der Halteeinrichtung und/oder an der Sägeschablone mindestens eine Befestigungselementaufnahme für eine Knochenbefestigungseinrichtung angeordnet oder ausgebildet ist. Beispielsweise kann die mindestens eine Befestigungselementaufnahme ausgebildet sein zum Aufnehmen eines oder mehrerer Befestigungselemente, beispielsweise in Form von Knochenschrauben oder Knochennägeln oder dergleichen.

Auf besonders einfache Weise lässt sich die mindestens eine Befestigungselementaufnahme ausbilden, wenn sie in Form einer Durchbrechung ausgebildet ist. Beispielsweise kann die Durchbrechung in Form einer Bohrung ausgebildet sein. Eine solche lässt sich sowohl an der Halteeinrichtung als auch an der Sägeschablone auf einfache Weise und in der gewünschten Genauigkeit ausbilden.

Vorteilhafterweise weist die Halteeinrichtung mindestens zwei Befestigungselementaufnahmen auf. Vorzugsweise verlaufen die Längsachsen der mindestens zwei Befestigungselementaufnahmen parallel oder im Wesentlichen parallel zueinander. Alternativ können die Längsachsen auch gegeneinander geneigt verlaufen. Durch die mindestens zwei Befestigungselementaufnahmen lässt sich jeweils mindestens ein Befestigungselement hindurchführen, um die Halteeinrichtung definiert und stabil an einem Knochen mindestens temporär festzu legen.

Ferner kann es vorteilhaft sein, wenn die Sägeschablone mindestens zwei Befestigungselementaufnahmen aufweist, deren Längsachsen gegeneinander geneigt verlaufen. Optional können die Längsachsen auch parallel zueinander verlaufen. In jedem Fall ermöglichen es die mindestens zwei Befestigungselementaufnahmen der Sägeschablone, diese ebenfalls definiert zumindest temporär an einem Knochen festzulegen, beispielsweise mit dafür geeigneten Befestigungselementen.

Für eine besonders stabile Ausgestaltung der Führungsvorrichtung ist es günstig, wenn die Sägeschablone einen Sägeschablonengrundkörper umfasst, an welchem der Führungsschlitz angeordnet oder ausgebildet ist.

Gemäß einer weiteren bevorzugten Ausführungsform der Erfindung kann vorgesehen sein, dass die Führungseinrichtung eine Positioniereinrichtung umfasst, welche mit der Halteeinrichtung in einer Positionierstellung gekoppelt ist. Mit der Positioniereinrichtung, die mit der Halteeinrichtung in der Positionierstellung gekoppelt ist, lässt sich die Halteeinrichtung einfach und definiert am Knochen positionieren. Optional kann die Positioniereinrichtung von der Halteeinrichtung vollständig abtrennbar sein, so dass sie nur für die Positionierung der Halteeinrichtung am Knochen zum Einsatz kommt, bei der Ausführung mindestens eines Sägeschnitts jedoch vollständig entfernt werden kann.

Vorteilhaft ist es, wenn die Positioniereinrichtung mindestens ein Knochenanlageelement mit einer Knochenanlagefläche und mindestens ein erstes Kupplungselement einer Kupplungseinrichtung umfasst, welches mindestens eine erstes Kupplungselement in der Positionierstellung mit mindestens einem zweiten Kupplungselement der Kupplungseinrichtung, welches an der Halteeinrichtung angeordnet oder ausgebildet ist, kraft- und/oder formschlüssig in Eingriff steht. Eine solche Kupplungseinrichtung ermöglicht es insbesondere, die Positioniereinrichtung und die Halteeinrichtung miteinander zu verbinden und gegebenenfalls auch wieder voneinander zu lösen.

Auf besonders einfache und schnelle Weise lassen sich die Positioniereinrichtung und die Halteeinrichtung miteinander mindestens temporär kuppeln, wenn die Kupplungseinrichtung in Form einer Rast- und/oder Schnappverbindungseinrichtung ausgebildet ist. Beispielsweise kann so die Positioniereinrichtung erfasst und mit der Halteeinrichtung gekoppelt werden. Insbesondere kann die Positioniereinrichtung auf die Halteeinrichtung aufgeclipst werden.

Besonders einfach und kompakt ausbilden lässt sich die Kupplungseinrichtung, wenn das mindestens eine zweite Kupplungselement in Form eines zylindrischen Kupplungskörpers ausgebildet ist und wenn das mindestens eine erste Kupplungselement in Form einer zum Kupplungskörper korrespondierenden Kupplungsaufnahme ausgebildet ist. Insbesondere kann der zylindrische Kupplungskörper das erste Koppelelement tragen oder umfassen. So ist es insbesondere möglich, die Halteeinrichtung und die Positioniereinrichtung miteinander zu kuppeln, so dass das erste Koppelelement zumindest temporär beiden Einrichtungen zugeordnet ist.

Die nachfolgende Beschreibung einer bevorzugten Ausführungsform der Erfindung dient im Zusammenhang mit der Zeichnung der näheren Erläuterung. Es zeigen:
- Figur 1:: eine perspektivische Darstellung einer an einen Knochen angelegten Positioniereinrichtung einer medizinischen Führungsvorrichtung vor dem in Eingriff Bringen mit einer Halteeinrichtung;

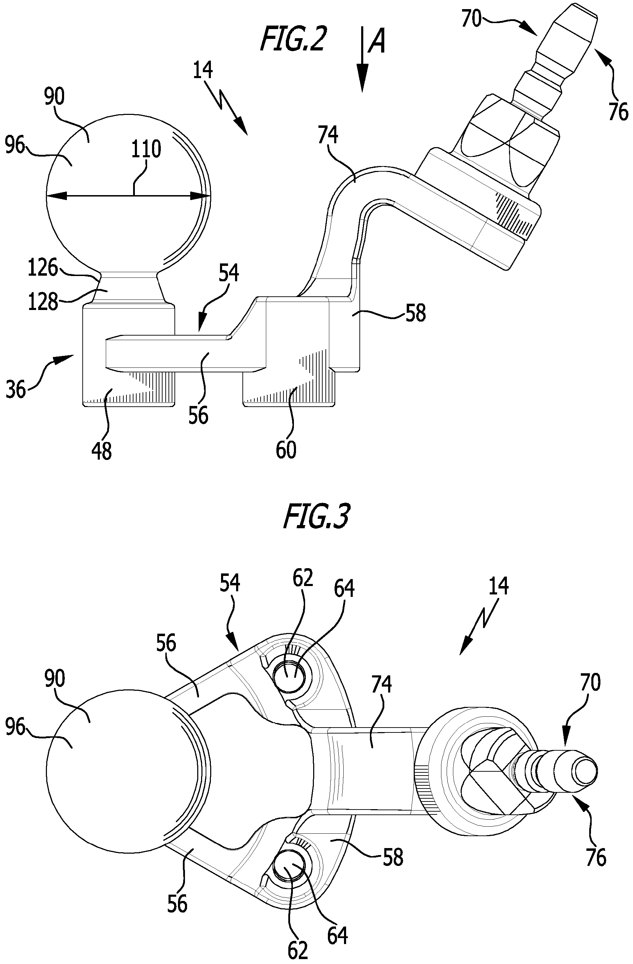

- Figur 2:: eine Seitenansicht der Halteeinrichtung aus Figur 1;
- Figur 3:: eine Ansicht der Halteeinrichtung aus Figur 2 in Richtung des Pfeils A;
- Figur 4:: eine schematische perspektivische Ansicht der miteinander gekoppelten Positioniereinrichtung und Halteeinrichtung vor dem Festlegen der Halteeinrichtung mittels zweier Knochenschrauben am Knochen;

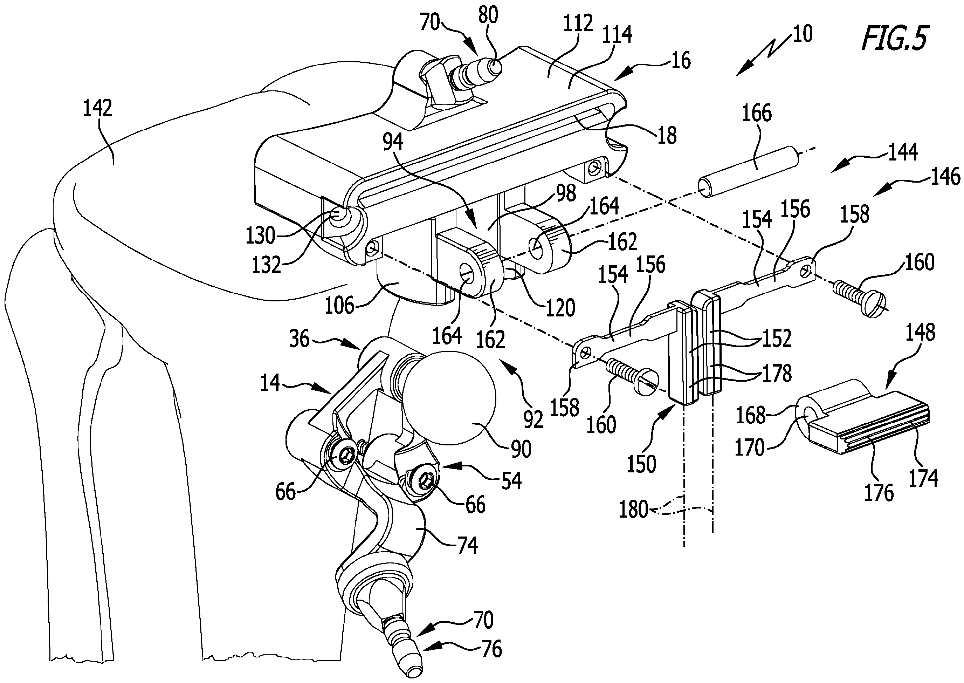

- Figur 5:: eine schematische perspektivische Ansicht der am Knochen festgelegten Halteeinrichtung mit einer Explosionsdarstellung einer Sägeschablone;

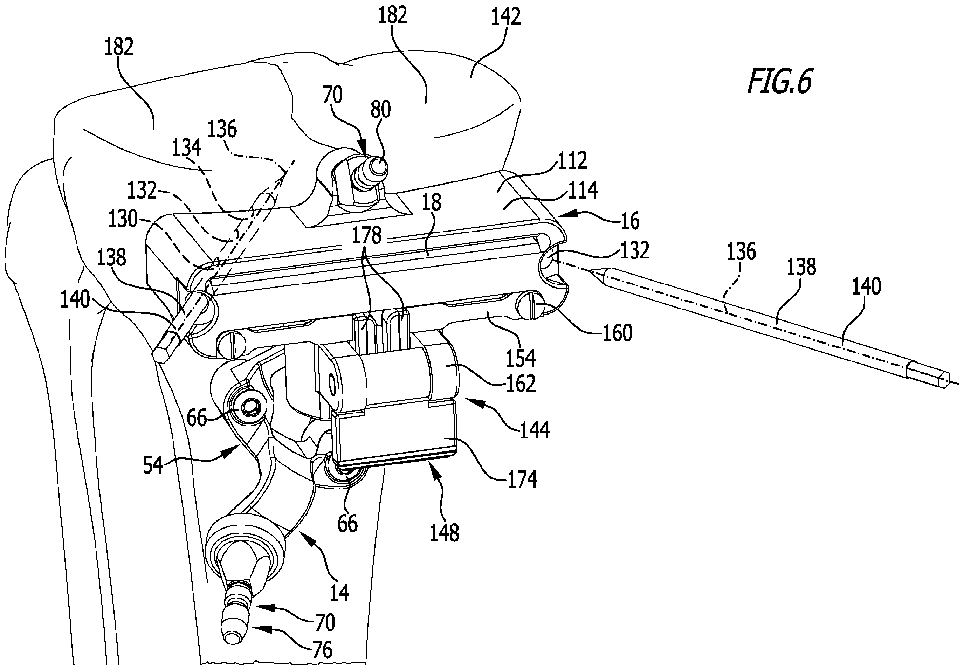

- Figur 6:: eine schematische perspektivische Darstellung einer am Knochen festgelegten Halteeinrichtung mit daran gekoppelter Sägeschablone in der Sägestellung beim Festlegen der Sägeschablone am Knochen mittels zweier Knochenpins;
- Figur 7:: eine schematische perspektivische Ansicht der am Knochen festgelegten Halteeinrichtung mit daran gekoppelter Sägeschablone in der Justierstellung, wobei sowohl die Sägeschablone als auch die Halteeinrichtung jeweils mit einer Referenzierungseinrichtung verbunden sind;

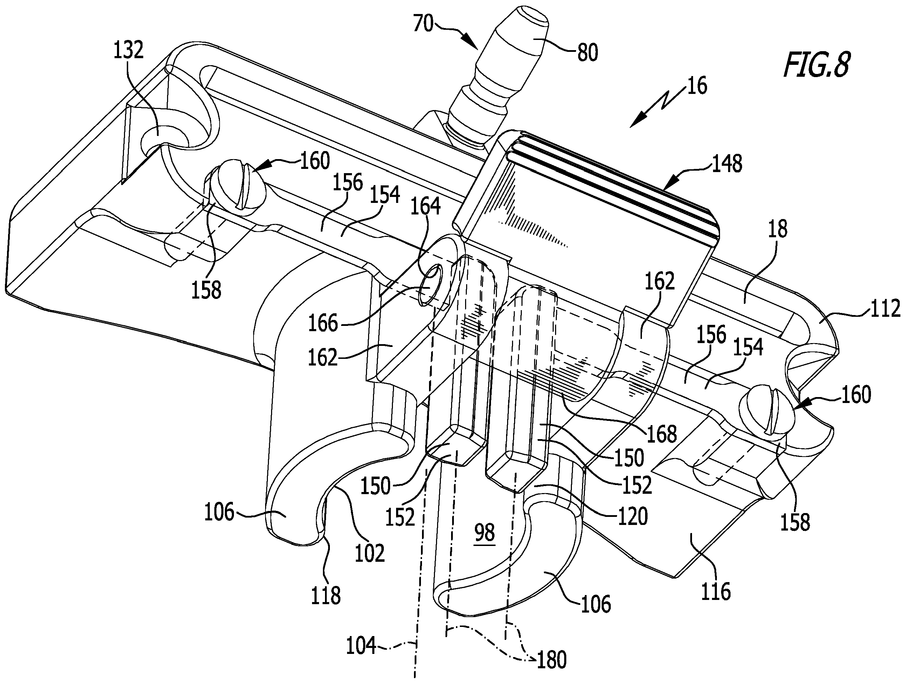

- Figur 8:: eine perspektivische, teilweise durchbrochene Ansicht der Sägeschablone in der Justierstellung;

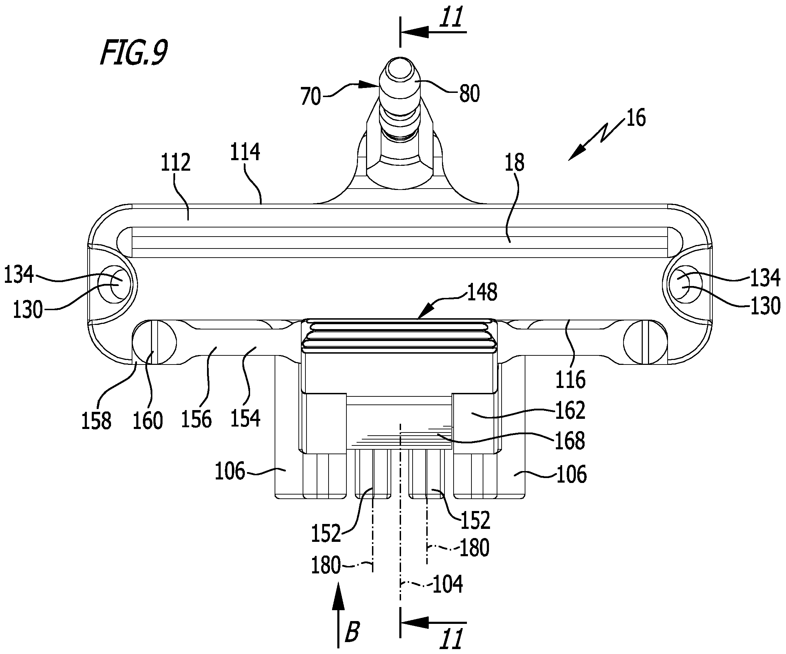

- Figur 9:: eine Draufsicht auf die Sägeschablone aus Figur 8;

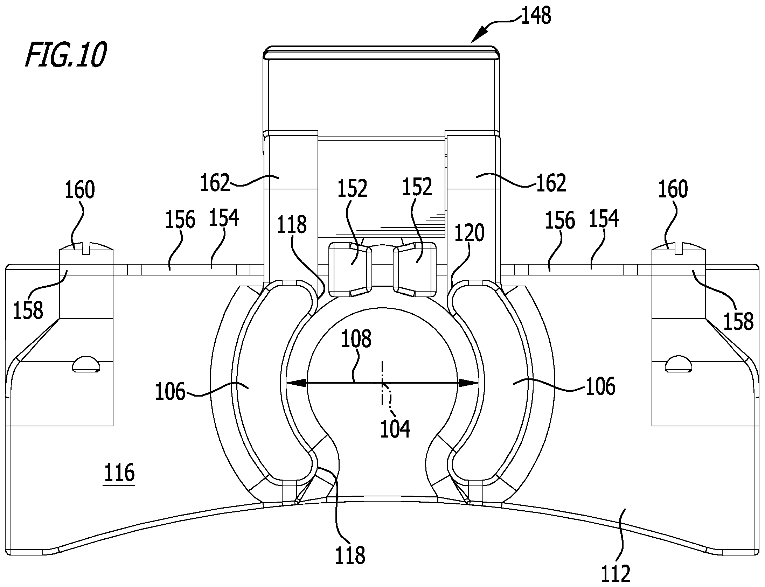

- Figur 10:: eine Ansicht der Sägeschablone aus Figur 9 in Richtung des Pfeils B;
- Figur 11:: eine Schnittansicht längs Linie 11-11 in Figur 9, wobei die medizinische Führungsvorrichtung die Justierstellung einnimmt;

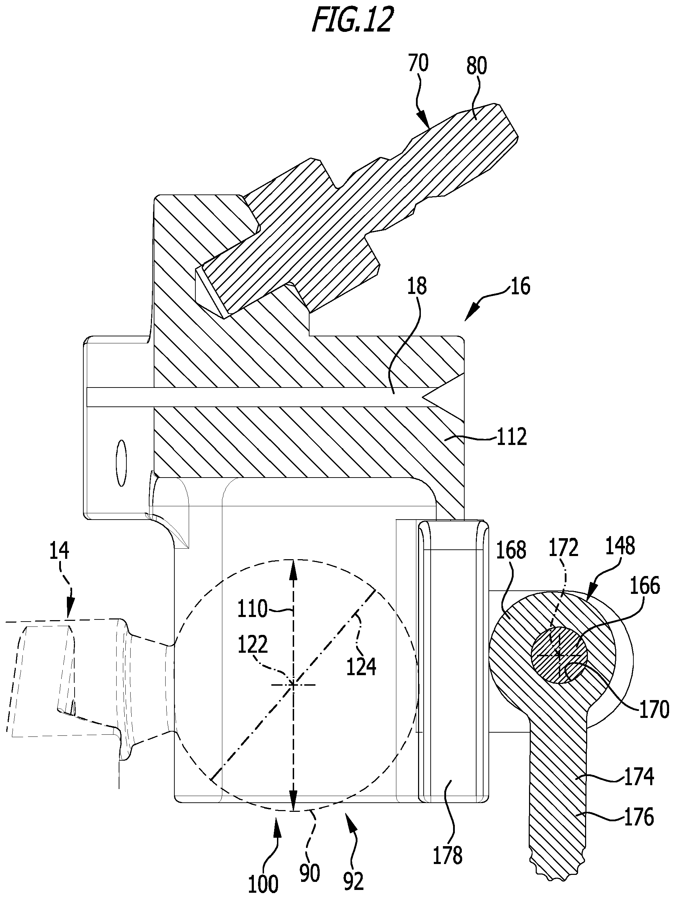

- Figur 12:: eine Schnittansicht ähnlich Figur 11, wobei die medizinische Führungsvorrichtung Sägestellung einnimmt;
- Figur 13:: eine teilweise durchbrochene perspektive Ansicht der Halteeinrichtung mit einem daran angekoppelten zweiten Ausführungsbeispiel einer Sägeschablone in der Sägestellung; und
- Figur 14:: eine teilweise Explosionsdarstellung der Anordnung aus Figur 13.

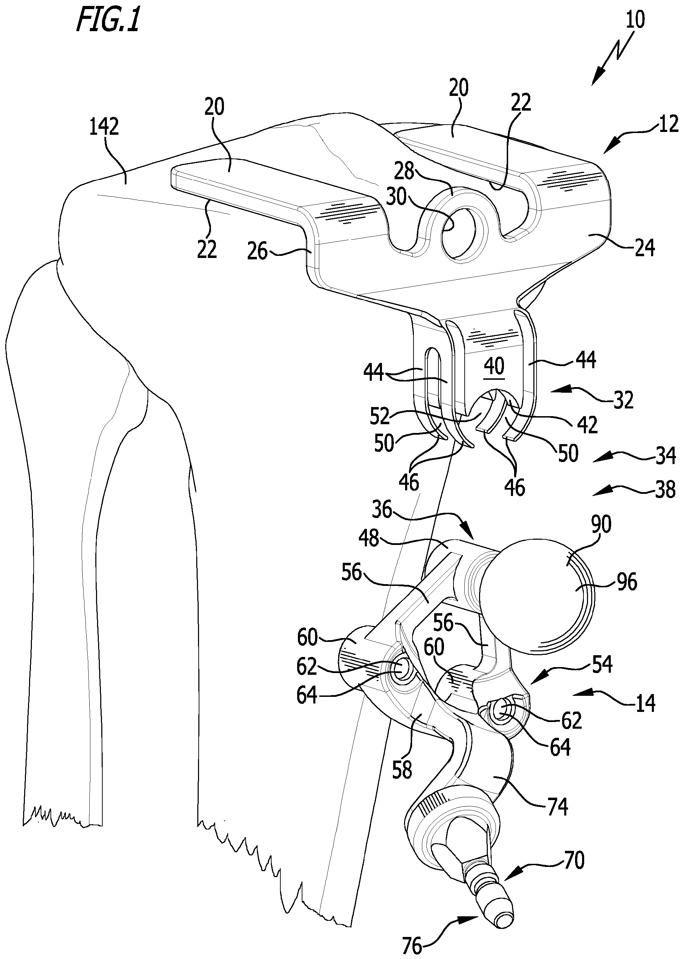

In Figur 1 ist schematisch ein Teil einer insgesamt mit dem Bezugszeichen 10 bezeichneten medizinischen Führungsvorrichtung dargestellt, und zwar eine Positioniereinrichtung 12 und eine Halteeinrichtung 14. Ferner umfasst die medizinische Führungsvorrichtung eine Sägeschablone 16 mit einem Führungsschlitz für ein Sägeblatt, beispielsweise einer oszillierenden Säge.

Die Positioniereinrichtung 12 umfasst zwei plattenförmige Knochenanlageelemente 20, welche jeweils eine Knochenanlagefläche 22 definieren. Die beiden Knochenanlageflächen 22 definieren eine gemeinsame Anlageebene.

Die Knochenanlageelemente 20 bilden mit einem Querträger 24 einen im Wesentlichen U-förmigen Anlagekörper 26. Der Querträger 24 ist aus einem Flachmaterial geformt. Die beiden Knochenanlageelemente 20 sind einstückig mit dem Querträger 24 ausgebildet und durch Umbiegen um 90° geformt.

Der Querträger 24 weist ferner eine in Richtung auf die von den Knochenanlageflächen 22 definierte Ebene hin vorspringenden, im Wesentlichen halbrund scheibenförmigen Haltelappen 28 auf, der mit einer kreisförmigen Durchbrechung 30 versehen ist.

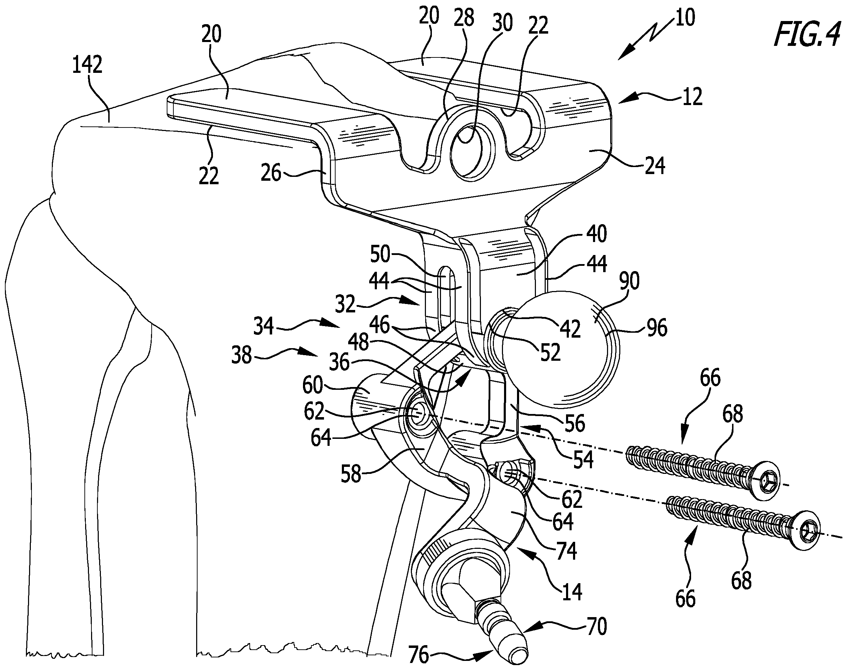

Vom Querträger 24 steht ferner in einer Richtung vom Haltelappen 28 weg weisend ein erstes Kupplungselement 32 einer insgesamt mit dem Bezugszeichen 34 bezeichneten Kupplungseinrichtung ab welches mit einem zweiten Kupplungselement 36, welches an der Halteeinrichtung 14 angeordnet oder ausgebildet ist, in einer Positionierstellung, wie sie beispielhaft in Figur 4 dargestellt ist, kraft- und/oder formschlüssig in Eingriff steht.

Die Kupplungseinrichtung 34 ist in Form einer Rast- und/oder Schnappverbindungseinrichtung 38 ausgebildet. Das erste Kupplungselement 32 umfasst zwei flache, parallel zum Querträger 24 abstehende Haltekörper 40, die jeweils eine vom jeweiligen Haltekörper 40 weg weisend konkav gekrümmte Anlagefläche 42 aufweisen, welche einen Ausschnitt einer hohlzylindrischen Fläche definiert.

Quer zu den Haltekörpern 40 sind diese seitlich umgebend zwei Blattfederelemente 44 an den Querträger 24 mit jeweils zwei, aufeinander zu weisend gekrümmten Enden 46 angeformt.

Die Anlageflächen 42 sowie die Enden 46 definieren im Wesentlichen eine hohlzylindrische Kontur zur Aufnahme des zweiten Kupplungselements 36, welches in Form eines geraden Kreiszylinders 48 ausgebildet ist. Die Blattfederelemente 44 definieren jeweils einen Spalt 50 zwischen den Enden 46.

Das zweite Kupplungselement 36 ist somit in Form eines zylindrischen Kupplungskörpers ausgebildet, das erste Kupplungselement 32 in Form einer zum Kreiszylinder 48 korrespondierenden Kupplungsaufnahme 52.

Die Halteeinrichtung 14 umfasst einen Halteeinrichtungsgrundkörper 54, welcher den Kreiszylinder 48 umfasst. Von diesem erstrecken sich zwei Schenkel 56 senkrecht zu einer Längsachse des Kreiszylinders 48 und gegeneinander abgewinkelt um einen Innenwinkel von etwa 60° zu einem schwach konvex vom Kreiszylinder 48 weg weisend gekrümmten Querträger 58.

Die Schenkel 56 und der Querträger 58 sind jeweils über einen Zylinderkörper 60 miteinander verbunden, welcher parallel zu einer Längsachse desselben mit einer Bohrung 62 versehen ist. Jede Bohrung definiert eine Befestigungselementaufnahme 64, durch die jeweils ein Befestigungselement 66 mindestens teilweise hindurchführbar ist. In Figur 4 sind als Befestigungselemente 66 beispielhaft zwei Knochenschrauben 68 dargestellt, deren mit einem Außengewinde versehenen Schäfte einen Außendurchmesser aufweisen, welcher an einen Innendurchmesser der Befestigungselementaufnahmen 64 angepasst ist.

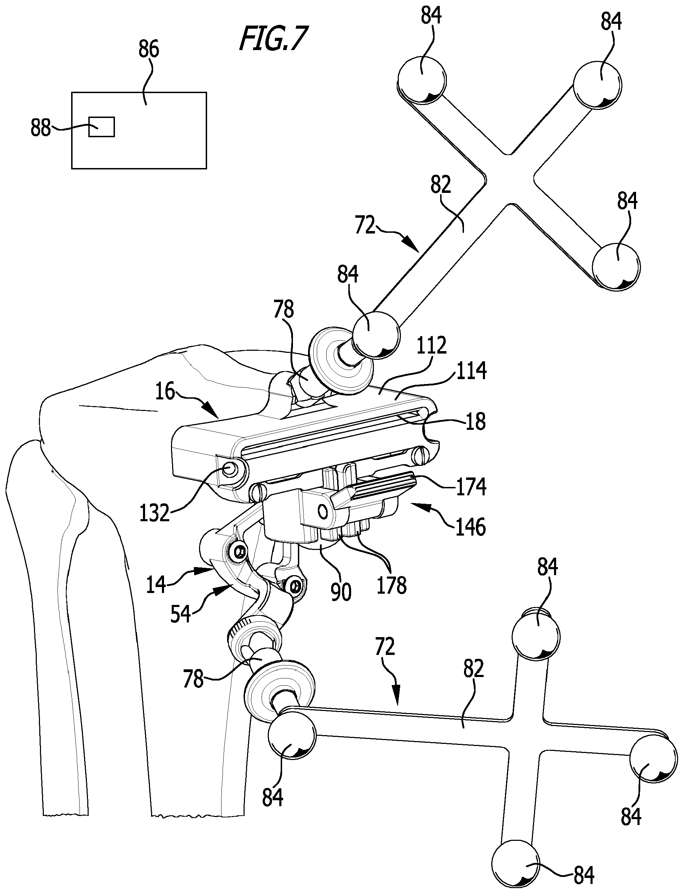

An der Halteeinrichtung 14 ist ferner eine Schnittstelle 70 für eine medizinische Referenzierungseinrichtung 72 angeordnet. Sie ist an einem Haltebügel 74 angeordnet beziehungsweise ausgebildet, welcher mit dem Querträger 58 verbunden ist. Die Schnittstelle 70 ist in Form eines ersten Verbindungselements 76 ausgebildet, welches korrespondierend zu einem zweiten Verbindungselement 78 der Referenzierungseinrichtung 72 ausgebildet ist. In einer Verbindungsstellung, wie sie schematisch in Figur 7 dargestellt ist, stehen die Verbindungselemente 76 und 78 miteinander in Eingriff. Sie können optional außer Eingriff gebracht werden und nehmen dann eine Reinigungsstellung ein.

Das erste Verbindungselement 76 ist in Form eines Verbindungsvorsprungs 80 ausgebildet, das zweite Verbindungselement 78 in Form einer zum Verbindungsvorsprung 80 korrespondierenden Verbindungsaufnahme. Alternativ kann das erste Verbindungselement 76 auch in Form einer Verbindungsaufnahme ausgebildet sein, die mit einem korrespondierenden Verbindungsvorsprung an der Referenzierungseinrichtung 72 in der Verbindungsstellung kraft- und/oder formschlüssig in Eingriff steht.

Die Referenzierungseinrichtung 72 umfasst einen im Wesentlichen kreuzförmigen Träger 82, welcher jeweils an freien Enden ein Markerelement 84 trägt. Die insgesamt vier Markerelemente 84 können wahlweise in Form passiver Markerelemente, welche elektromagnetische Strahlung oder Ultraschall reflektieren, oder in Form aktiver Markerelemente ausgebildet sein, welche elektromagnetische Strahlung oder Ultraschall aussenden.

Eine Position der Referenzierungseinrichtung 72 kann durch ein medizinisches Navigationssystem 86 bestimmt werden, welches mindestens einen Detektor 88 umfasst, mit welchem von den Markerelementen 84 reflektierte oder ausgesandte Strahlung detektiert werden kann.

Die Halteeinrichtung 14 trägt ferner ein erstes Koppelelement 90 einer insgesamt mit dem Bezugszeichen 92 bezeichneten Koppeleinrichtung zum Koppeln der Halteeinrichtung 14 und der Sägeschablone 16 miteinander. An der Sägeschablone 16 ist ein zweites Koppelelement 94 der Koppeleinrichtung 92 angeordnet.

Das erste Koppelelement 90 ist in Form einer Gelenkkugel 96 ausgebildet, das zweite Koppelelement 94 in Form einer Gelenkkugelaufnahme 98. So umfasst die Koppeleinrichtung 92 insgesamt ein Kugelgelenk 100.

Sind die ersten und zweiten Verbindungselemente 76 und 78 voneinander getrennt, nehmen sie die oben definierte Reinigungsstellung ein. Beispielsweise ist in Figur 1 das erste Verbindungselement 76 nicht mit dem zweiten Verbindungselement 78 gekoppelt.

Die Koppeleinrichtung 92 ist derart ausgebildet, dass die Sägeschablone 16 und die Halteeinrichtung 14 in einer Justierstellung relativ zueinander verschiebbar aneinander gelagert sind. Um dies zu erreichen, definiert die Gelenkkugelaufnahme 96 eine hohlzylindrische oder im Wesentlichen hohlzylindrische Innenkontur 102. Ein Innendurchmesser 108, welcher durch die Innenkontur 102 vorgegeben ist, ist an einen Außendurchmesser 110 der Gelenkkugel 96 angepasst, sodass die Gelenkkugel 96 parallel zu einer vom zweiten Koppelelement 94 definierten Koppelelementlängsachse 104 in der Justierstellung relativ tief zueinander verschiebbar ist.

Die Gelenkkugelaufnahme 98 umfasst zwei einander im Wesentlichen diametral gegenüberliegende hohlzylindrische Wandabschnitte 106, welche den Innendurchmesser 108 definieren, welcher dem Außendurchmesser 110 der Gelenkkugel 96 entspricht oder unwesentlich kleiner ist.

Die Wandabschnitte 106 stehen senkrecht von einem im Wesentlichen quaderförmigen Sägeschablonengrundkörper, nachfolgend als Sägeblock 112 bezeichnet, der Sägeschablone 16 ab. Der Sägeblock 112 umfasst ferner den Führungsschlitz 18, welcher parallel zu einer Oberseite 114 des Sägeblocks 112 denselben durchsetzt. Zwischen den beiden Wandabschnitten 106, die von einer Unterseite 116 des Sägeblocks 112 abstehen, sind zwei einander diametral gegenüberliegende Spalte 118 ausgebildet, von denen einer einen Koppelspalt 120 bildet.

Die beiden Wandabschnitte 106 definieren eine Linearführung für die Gelenckugel 96, welche zudem zwischen den Wandabschnitten 106 um ihren einen Drehpunkt 122 des Kugelgelenks 100 definierenden Mittelpunkt rotierbar ist. Dies ermöglicht es insbesondere, die Sägeschablone 16 nicht nur parallel zur Koppelelementlängsachse 104 relativ zur Halteeinrichtung 14 zu verschieben, sondern relativ zu dieser auch um beliebig viele, durch den Drehpunkt 122 verlaufende Schwenkachsen 124 zu verschwenken beziehungsweise um den Drehpunkt 122 zu rotieren. Insgesamt ist so eine Relativbewegung zwischen der Halteeinrichtung 14 und der Sägeschablone 16 in mindestens vier Bewegungsfreiheitsgraden möglich.

Die Koppeleinrichtung 92 ist somit derart ausgebildet, dass die Sägeschablone und die Halteeinrichtung 14 in der Justierstellung relativ zueinander um eine Schwenkachse verschwenkbar aneinander gelagert sind.

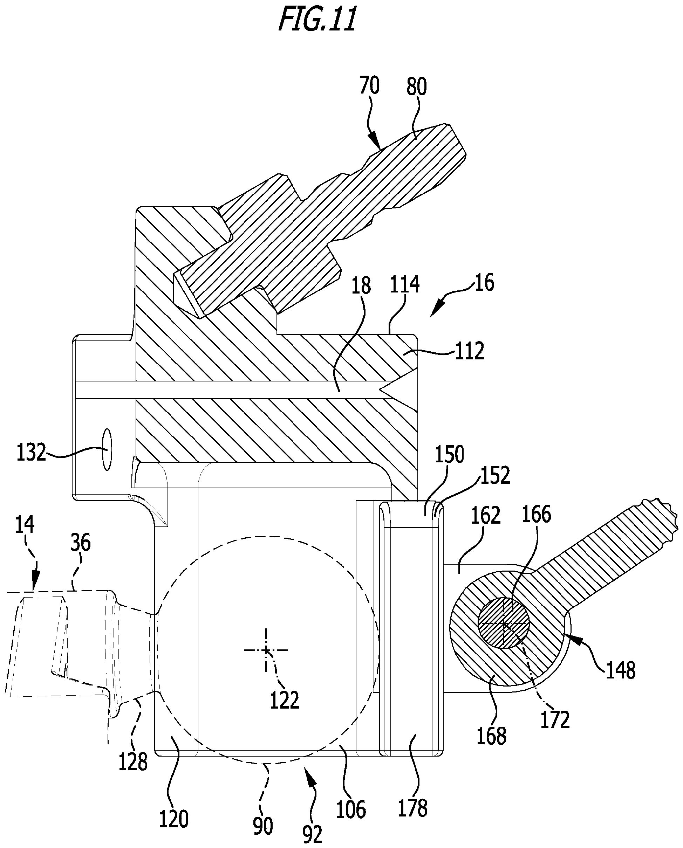

Die ersten und zweiten Koppelelemente 90 und 94 können auf einfache Weise dadurch in Eingriff gebracht werden, dass zwischen dem ersten Koppelelement 90 und dem Halteeinrichtungsgrundkörper 54, insbesondere dem zweiten Kopplungselement 36, eine Einschnürung 126 beziehungsweise ein Verbindungssteg 128 ausgebildet ist, welche den Koppelspalt 120 der Gelenkkugelaufnahme 98 in der Justierstellung durchsetzen. Dies ist beispielsweise in Figur 11 schematisch dargestellt.

Der Koppelspalt 120 ist vorzugsweise breiter als eine äußere Abmessung des Verbindungsstegs 128, sodass die Sägeschablone 16 relativ zur Halteeinrichtung 14 insbesondere um die Koppelelementlängsachse 104 verdrehbar ist.

An der Sägeschablone 16, nämlich an deren Sägeblock 112, sind ferner zwei Befestigungselementaufnahmen 130 in Form von nicht parallel zueinander orientierten Durchbrechungen 132 ausgebildet. Die Durchbrechungen 132 sind in Form von Bohrungen 134 ausgebildet, deren Längsachsen 136 nicht parallel, vorzugsweise windschief relativ zueinander verlaufen. Durch die Befestigungselementaufnahmen 130 können Knochenbefestigungseinrichtungen 138 in Form von Knochenpins 140 hindurchgeführt werden, um den Sägeblock 112 in definierter Weise fest an einem Knochen 142 zu fixieren.

Auf der Oberseite 114 ist eine weitere Schnittstelle 70 für eine Referenzierungseinrichtung 72 angeordnet, mit einem Verbindungsvorsprung 80, dessen Längsachse quer zu einer von der Oberseite 114 definierten Ebene verläuft. Die Schnittstelle 70 ermöglicht es, die Sägeschablone 16 mit einer weiteren Referenzierungseinrichtung 72 zu koppeln, wie dies schematisch in Figur 7 dargestellt ist. Dies ermöglicht es insbesondere, eine Position der Sägeschablone 16 im Raum durch das Navigationssystem 86 zu ermitteln. Insbesondere können so auch Relativbewegungen und -orientierungen zwischen den Referenzierungseinrichtungen 72, die einerseits mit der Halteeinrichtung 14 und andererseits mit der Sägeschablone 16 verbunden sind, hochpräzise ermittelt werden.

Bislang wurde die Koppeleinrichtung 92 nur so beschrieben, dass eine Relativbewegung der Halteeinrichtung 14 und der Sägeschablone 16 zueinander in der Justierstellung möglich ist. Die Führungsvorrichtung 10 umfasst jedoch ferner auch eine Festelleinrichtung 144 zum kraft- und/oder formschlüssigen Halten der Halteeinrichtung 14 und der Sägeschablone 16 in einer Sägestellung. In dieser sind die Halteeinrichtung 14 und die Sägeschablone 16 unbeweglich aneinander gehalten.

Die Festelleinrichtung 144 kann insbesondere in Form einer Rast- und/oder Schnappverbindung ausgebildet sein. In den Figuren ist beispielhaft eine Feststelleinrichtung 144 in Form einer Klemmeinrichtung 146 ausgebildet.

Die Feststelleinrichtung 144 umfasst ein erstes Feststellelement 148, welches in der Sägestellung das erste Koppelelement 90 gegen das zweite Koppelelement 94 drückt. Bei dem in den Figuren dargestellten Ausführungsbeispiel erfolgt die Übertragung einer Klemmkraft indirekt vom ersten Feststellelement 148 auf das erste Koppelelement 90. Die Feststelleinrichtung 144 umfasst hierfür zwei zweite Feststellelemente 150 in Form von langgestreckten, quaderförmigen Stäben 152, welche jeweils über ein Federelement 154 in Form einer Blattfeder 156 am Sägeblock 112 festgelegt sind. Hierfür ist ein freies Ende 158 jeder Blattfeder durchbohrt und mit einer Schraube 160 an den Sägeblock 112 angeschraubt.

Die Blattfedern 156 tragen an ihrem dem Ende 158 abgewandten Ende die Stäbe 152. Die Blattfederelemente 156 sind aufeinander zuweisend ausgerichtet, wobei die Stäbe 152 quer, im Wesentlichen senkrecht zu von den Federelementen 154 definierten Längsachsen von diesen abstehen. Die zweiten Feststellelemente 150 sind parallel zueinander im Spalt 118 angeordnet, welcher dem Koppelspalt 120 gegenüber liegt. Die Stäbe 152 definieren so einen Teil der Innenkontur 102 der Gelenkkugelaufnahme 98.

Von den Wandabschnitten 106 steht jeweils ein im Wesentlichen quaderförmiger Lagerkörper 162 ab, welcher mit einer Bohrung 164 versehen ist. Die Bohrungen 164 sind koaxial zueinander ausgerichtet und von einem zylindrischen Lagerstift 166 durchsetzt.

Auf dem Lagerstift 166 ist ein Exzenterkörper 168 in Form eines sich zwischen den Lagerkörpern 162 erstreckenden Zylinders verschwenkbar gelagert. Der Exzenterkörper 168 ist von einer Bohrung 170 durchsetzt, deren Längsachse exzentrisch zu einer Längsachse des Exzenterkörpers 168 verläuft. Der Exzenterkörper 168 ist so um eine vom Lagerstift 166 definierte Exzenterschwenkachse 172 verschwenkbar. Die Exzenterschwenkachse verläuft quer zur Koppelelementlängsachse 104 und parallel zum Führungsschlitz 18.

Das erste Feststellelement 148 umfasst ferner ein Betätigungsglied 174, und zwar in Form eines vom Exzenterkörper 168 abstehend ausgebildeten, flachen quaderförmigen Vorsprungs 176.

Wird das zweite Feststellelement 150 aus der in Figur 11 dargestellten Justierstellung in die Sägestellung verschwenkt, drückt der Exzenterkörper 168 zunehmend gegen die Andrückkörper 178 definierende Stäbe 152, deren Andrückkörperlängsachsen 180 parallel zur Koppelelementlängsachse 104 verlaufen. Dabei werden die Andrückkörper 178 gegen die Gelenkkugel 96 gedrückt.

Die Federelemente 154 sind so ausgebildet, dass die Andrückkörper 178 bereits ohne Einwirkung des Exzenterkörpers 168 gegen die Gelenkkugel 96 vorgespannt sind. So ist die Sägeschablone 16 bereits ohne Wirkung des Exzenterkörpers 168 zumindest mit einer definierten Klemmkraft klemmend an der Halteeinrichtung 14 gehalten. Durch entsprechende Verschwenkung des zweiten Feststellelements 150 kann die Andrückkraft, die über die Andrücckörper 178 auf die ersten und zweiten Koppelelemente 90 und 94 übertragen wird, stufenlos erhöht werden bis die Sägeschablone 16 und die Halteeinrichtung 14 nicht mehr relativ zueinander bewegbar sind.

Die Verwendung der medizinischen Führungsvorrichtung 10 wird nachfolgend kurz erläutert.

Zunächst wird die Positioniereinrichtung 12 mit den Knochenanlageflächen 22 an entsprechende Knochenflächen 182 des Knochens 142, beispielweise an in Richtung auf einen nicht dargestellten Femurknochen hin weisenden Knochenflächen 182 einer Tibia, angelegt. Dabei kann die Halteeinrichtung 14 bereits mit der Positioniereinrichtung 12 gekoppelt sein. Ist sie dies nicht, wird das zweite Kupplungselement 36 in die Kupplungselementaufnahme 52 eingeclipst. Die Spalte 50 dienen dabei insbesondere dazu, die Schenkel 56 aufzunehmen. Die Halteeinrichtung 14 kann, wenn sie mit der Positioniereinrichtung 12 gekuppelt ist, um die vom Kreiszylinder 48 definierte Längsachse relativ zur Positioniereinrichtung 12 verdreht werden.

In einem nächsten Schritt wird die Halteeinrichtung 14 mit den beiden Knochenschrauben 68 am Knochen 142 fixiert und danach die Positioniereinrichtung 12 wieder von der Halteeinrichtung 14 gelöst. In einem nächsten Schritt wird die Sägeschablone 16 mit der Halteeinrichtung 14 gekoppelt. Dazu wird die Gelenkkugelaufnahme 98 über die Gelenkkugel 96 geführt, sodass der Verbindungssteg 128 den Koppelspalt 120 durchsetzt.

Werden sowohl die Halteeinrichtung 14 als auch die Sägeschablone 16 mit einer Referenzierungseinrichtung 72 verbunden, können deren Positionen und/ oder Orientierungen im Raum durch das Navigationssystem 86 bestimmt werden. Dies gestattet es, die Sägeschablone 16 zur Ausbildung eines Sägeschnitts am Knochen 142 wie gewünscht und hochpräzise auszurichten. Dies erfolgt durch eine entsprechende Verschiebe- und /oder Verschwenkbewegung der Sägeschablone 16 relativ zur Halteeinrichtung 14 in der Justierstellung. Nimmt die Sägeschablone 16 die gewünschte Position und Orientierung ein, wird durch Verschwenken des ersten Feststellelements 148 der Feststelleinrichtung 144 die Sägeschablone16 unbeweglich an der Halteeinrichtung 14 gesichert.

Um eine Bewegung der Sägeschablone 16 relativ zum Knochen 142 zu verhindern, wenn ein Sägeblatt durch den Führungsschlitz 18 hindurchgeführt wird, können optional die Knochenpins 140 durch die Bohrungen 134 hindurch in den Knochen 142 eingetrieben werden.

Nach Bearbeitung des Knochens 142 mit einer vorzugsweise oszillierenden Knochensäge wird die Sägeschablone 16 wieder vom Knochen 142 gelöst, indem die Knochenpins 140 entfernt werden. Die Sägeschablone 16 kann dann von der Halteeinrichtung 14 gelöst werden, indem die Feststelleinrichtung 144 wieder von der Sägestellung in die Justierstellung überführt wird, durch Zurückverschwenken des ersten Feststellelements 148.

Nach Abnehmen der Sägeschablone 16 von der Halteeinrichtung 14 werden die Knochenschrauben 68 wieder aus dem Knochen 142 entfernt und die Halteeinrichtung 14 abgenommen.

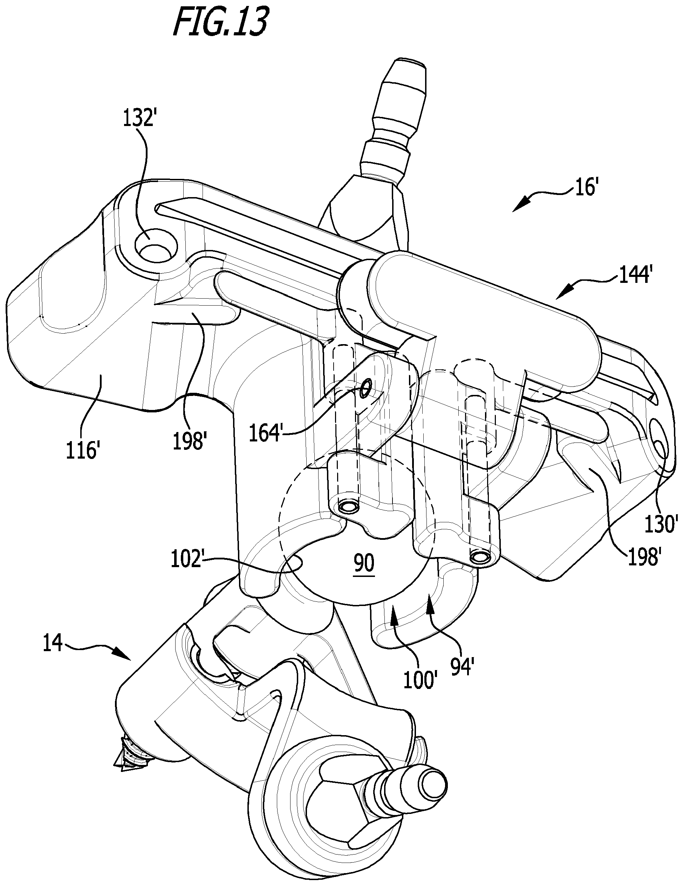

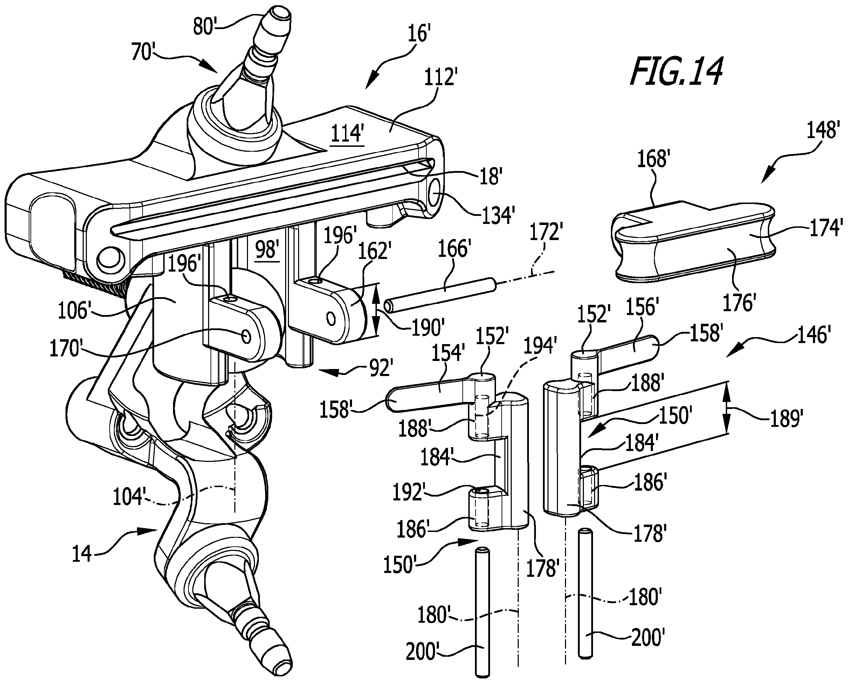

In den Figuren 13 und 14 ist beispielhaft ein zweites, insgesamt mit dem Bezugszeichen 16' bezeichnetes Ausführungsbeispiel einer Sägeschablone mit der Halteeinrichtung 14 in der Justierstellung gekoppelt dargestellt.

Die Sägeschablone 16' unterscheidet sich von der Sägeschablone 16 in ihrem grundsätzlichen Aufbau nicht. Es werden daher nachfolgend nur die wesentlichen Unterschiede der Sägeschablone 16' im Vergleich zur Sägeschablone 16 kurz erläutert. Dabei werden zur Kennzeichnung von Teilen der Sägeschablone 16', die mit Teilen der Sägeschablone 16 übereinstimmen oder diesen entsprechen, Bezugszeichen mit nachgestelltem Anstrich verwendet.

Die Festelleinrichtung 144' der Sägeschablone 16' umfasst ein erstes Feststellelement 148', welches in der Sägestellung das erste Koppelelement 90 gegen das zweite Koppelelement 94' drückt. Auch bei der Sägeschablone 16' erfolgt die Übertragung einer Klemmkraft indirekt vom ersten Feststellelement 148' auf das erste Koppelelement 90. Die Feststelleinrichtung 144' umfasst hierfür ebenfalls zwei zweite Feststellelemente 150' in Form von langgestreckten, quaderförmigen Stäben 152', die auf voneinander weg weisenden Seiten 184' jeweils einen Lagerkörper 186' und einen Lagerkörper 188' tragen. Ein Abstand 189' der an einem Stab 152' ausgebildeten Lagerböcke 186' und 188' voneinander entspricht einer Höhe 190' der Lagerkörper 162'.

Der Lagerbock 186' ist zudem mit einer Durchgangsbohrung 192' versehen, der Lagerbock 188' mit einer in Richtung auf die Durchgangsbohrung 192' hin geöffneten Sacklochbohrung 194', die mit der Durchgangsbohrung 192' fluchtend ausgerichtet ist und parallel zu den Andrückkörperlängsachsen 180' verläuft.

Parallel zur Koppelelementlängsachse 104' sind die Lagerkörper 162' zusätzlich zu den Bohrungen 164' mit jeweils einer Bohrung 196' versehen, wobei Längsachsen derselben rechtwinklig zueinander verlaufen, sich aber nicht schneiden. Die Lagerböcke 188' ragen etwas über ein auf den Sägeblock 112' hin weisendes Ende der Stäbe 152' vor. In diesem Bereich ist an jeden Lagerbock 188' ein Federelement 154' in Form einer Blattfeder 156' angeformt.

Dort, wo bei der Sägeschablone 16 mit Innengewinde versehene Bohrungen zum in Eingriff Bringen mit den Schrauben 160 ausgebildet sind, sind bei der Sägeschablone 16' schräg in Richtung auf die Lagerböcke 188' hin weisende Vorsprünge 198' ausgebildet, welche als Widerlager für freie Enden 158'der Blattfedern 156' dienen.

Die freien Enden 158' sind, anders als die freien Enden 158 bei der Sägeschablone 16, nicht an den Vorsprüngen 198' fixiert. Um jedoch eine definierte Positionierung und Bewegung der Andrückkörper 178' zu erreichen, sind die Stäbe 152' schwenkbar mit den Lagerkörpern 162' gekoppelt. Hierfür dienen Lagerstifte 200', die mit ihrem einen Ende in die Sacklochbohrung 194' am Lagerbock 188' eingreifen und sowohl die Bohrung 196' am Lagerkörper 162' als auch die Durchgangsbohrung 192' am Lagerbock 186' durchsetzen. Somit definieren Längsachsen der Lagerstifte 200' Schwenkachsen, um welche die Andrückkörper 178' verschwenkbar sind.

Durch eine Verschwenkung des ersten Feststellelements 148' kann das erste Koppelelement 90 in der Gelenkkugelaufnahme 98' geklemmt werden. Dabei liegen die bereits in der Justierstellung federnd gegen die Gelenkkugel 96 vorgespannten Stäbe 192' klemmend am ersten Koppelelement 90 an.

Im Übrigen unterscheidet sich der konstruktive Aufbau der Sägeschablone 16' von der Sägeschablone 16 nur unwesentlich, so dass zur Funktionsweise der Sägeschablone 16' und insbesondere zur Vermeidung von Wiederholungen auf die obigen Ausführungen zur Funktionsweise der Sägeschablone 16 verwiesen werden kann.

### Bezugszeichenliste

- 10: Medizinische Führungsvorrichtung
- 12: Positioniereinrichtung
- 14: Halteeinrichtung
- 16, 16': Sägeschablone
- 18, 18': Führungsschlitz
- 19: Kupplungskörper
- 20: Knochenanlageelement
- 22: Knochenanlagefläche
- 24: Querträger
- 26: Anlagekörper
- 28: Haltelappen
- 30: Durchbrechung
- 32: erstes Kupplungselement
- 34: Kupplungseinrichtung
- 36: zweites Kupplungselement
- 38: Rast- und/oder Schnappverbindungseinrichtung
- 40: Haltekörper
- 42: Anlagefläche
- 44: Blattfederelemente
- 46: Ende
- 48: Kreiszylinder
- 50: Spalt
- 52: Kupplungsaufnahme
- 54: Halteeinrichtungsgrundkörper
- 56: Schenkel
- 58: Querträger
- 60: Zylinderkörper
- 62: Bohrung
- 64: Befestigungselementaufnahme
- 66: Befestigungselement
- 68: Knochenschraube
- 70, 70': Schnittstelle
- 72: Referenzierungseinrichtung
- 74: Haltebügel
- 76: erstes Verbindungselement
- 78: zweites Verbindungselement
- 80, 80': Verbindungsvorsprung
- 82: Träger
- 84: Markerelement
- 86: Navigationssystem
- 88: Detektor
- 90: erstes Koppelelement
- 92, 92': Koppeleinrichtung
- 94, 94': zweites Koppelelement
- 96: Gelenkkugel
- 98, 98': Gelenkkugelaufnahme
- 100, 100': Kugelgelenk
- 102, 102': Innenkontur
- 104, 104': Koppelelementlängsachse
- 106, 106': Wandabschnitt
- 108: Innendurchmesser
- 110: Außendurchmesser
- 112, 112': Sägeblock
- 114, 114': Oberseite
- 116, 116': Unterseite
- 118: Spalt
- 120: Koppelspalt
- 122: Drehpunkt
- 124: Schwenkachse
- 126: Einschnürung
- 128: Verbindungssteg
- 130, 130': Befestigungselementaufnahme
- 132, 132': Durchbrechung
- 134, 134': Bohrung
- 136: Längsachse
- 138: Knochenbefestigungseinrichtung
- 140: Knochenpin
- 142: Knochen
- 144, 144': Feststelleinrichtung
- 146, 146': Klemmeinrichtung
- 148, 148': erstes Feststellelement
- 150, 150': zweites Feststellelement
- 152, 152': Stab
- 154, 154': Federelement
- 156, 156': Blattfeder
- 158, 158': Ende
- 160: Schraube
- 162, 162': Lagerkörper
- 164, 164': Bohrung
- 166, 166': Lagerstift
- 168, 168': Exzenterkörper
- 170, 170': Bohrung
- 172, 172': Exzenterschwenkachse
- 174, 174': Betätigungsglied
- 176, 176': Vorsprung
- 178, 178': Andrückkörper
- 180, 180': Andrückkörperlängsachse
- 182: Knochenfläche
- 184': Seitenfläche
- 186': Lagerbock
- 188': Lagerbock
- 189': Abstand
- 190': Höhe
- 192': Durchgangsbohrung
- 194': Sacklochbohrung
- 196': Bohrung
- 198': Vorsprung
- 200': Lagerstift

## Patentansprüche

1. Medizinische Führungsvorrichtung (10) zum Bearbeiten eines Knochens, welche Führungsvorrichtung (10) eine an einem Knochen festlegbare Halteeinrichtung (14) zum Festlegen der Führungsvorrichtung (10) am Knochen und eine Sägeschablone (16) mit einem Führungsschlitz (18) für ein Sägeblatt umfasst, wobei die Halteeinrichtung (14) und die Sägeschablone (16) in einer Justierstellung miteinander in Eingriff stehen und relativ zueinander verstellbar angeordnet sind, wobei die Halteeinrichtung (14) und die Sägeschablone (16; 16') von der Justierstellung in eine Sägestellung bringbar sind, in welcher sie relativ zueinander unbeweglich festgelegt sind, welche Führungsvorrichtung (10) eine Koppeleinrichtung (92) umfasst zum Koppeln der Halteeinrichtung (14) und der Sägeschablone (16; 16') in der Justierstellung und in der Sägestellung, wobei die Koppeleinrichtung (92) erste und zweite Koppelelemente (90, 94; 90, 94') umfasst, die einerseits an der Halteeinrichtung (14) und andererseits an der Sägeschablone (16; 16') angeordnet oder ausgebildet sind und in der Justierstellung miteinander in Eingriff stehen und in der Sägestellung kraft- und/oder formschlüssig aneinander gehalten sind, wobei das erste Koppelelement (90) in Form einer Gelenkkugel (96) ausgebildet ist, wobei das zweite Koppelelement in Form einer Gelenkkugelaufnahme (98) ausgebildet ist, wobei die Gelenkkugelaufnahme (98) eine hohlzylindrische oder im Wesentlichen hohlzylindrische Innenkontur (102) definiert und es ermöglicht, die darin gehaltene Gelenkkugel (96) nicht nur um einen von der Gelenkkugel (96) definierten Mittelpunkt zu rotieren, sondern die Gelenkkugel (96) relativ zur Gelenkkugelaufnahme (98) auch parallel zu einer von der Gelenkkugelaufnahme (98) definierten Längsachse (104) zu verschieben, **dadurch gekennzeichnet, dass** es die Koppeleinrichtung (92) ermöglicht, nach gewünschter Ausrichtung der Sägeschablone (16) die ersten und zweiten Koppelelemente (90, 94; 90, 94') von der Justierstellung in die Sägestellung zu überführen, um in dieser Sägestellung die Sägeschablone (16) in der definierten Ausrichtung zu halten.

2. Medizinische Führungsvorrichtung nach Anspruch 1, **dadurch gekennzeichnet, dass** an der Halteeinrichtung (14) und/oder an der Sägeschablone (16) eine Schnittstelle (70) für eine medizinische Referenzierungseinrichtung (72) angeordnet oder ausgebildet ist, deren Position und/ oder Orientierung im Raum durch ein medizinisches Navigationssystem (86) detektierbar ist und/oder dass an der Halteeinrichtung (14) und/ oder an der Sägeschablone (16) eine medizinische Referenzierungseinrichtung (72) angeordnet oder ausgebildet ist, deren Position und/oder Orientierung im Raum durch ein medizinisches Navigationssystem (86) detektierbar ist.

3. Medizinische Führungsvorrichtung nach Anspruch 2, **dadurch gekennzeichnet, dass**
a) die Schnittstelle (70) in Form eines ersten Verbindungselements (76) ausgebildet ist, welches korrespondierend zu einem zweiten Verbindungselement (78) der medizinischen Referenzierungseinrichtung (72) ausgebildet ist und dass das erste und das zweite Verbindungselement (76, 78) in einer Verbindungsstellung in Eingriff stehen und in einer Reinigungsstellung außer Eingriff stehen
und/oder
b) die medizinische Referenzierungseinrichtung (72) mindestens ein Markerelement trägt, dessen Position im Raum durch ein medizinisches Navigationssystem (86) detektierbar ist.

4. Medizinische Führungsvorrichtung nach einem der voranstehenden Ansprüche, **dadurch gekennzeichnet, dass**
a) die Koppeleinrichtung (92) derart ausgebildet ist, dass die Sägeschablone (16) und die Halteeinrichtung (14) in der Justierstellung relativ zueinander verschiebbar aneinander gelagert sind
und/oder
b) das zweite Koppelelement (94) eine Koppelelementlängsachse (104) definiert und dass die Sägeschablone (16) und die Halteeinrichtung (14) in der Justierstellung relativ zueinander parallel zur Koppelelementlängsachse (104) verschiebbar aneinander gelagert sind
und/oder
c) die Koppeleinrichtung (92) derart ausgebildet ist, dass die Sägeschablone (16) und die Halteeinrichtung (14) in der Justierstellung relativ zueinander um eine Schwenkachse (124) verschwenkbar aneinander gelagert sind.

5. Medizinische Führungsvorrichtung nach einem der voranstehenden Ansprüche, **dadurch gekennzeichnet, dass** die Koppeleinrichtung (92)
a) derart ausgebildet ist, dass die Sägeschablone (16) und die Halteeinrichtung (14) in der Justierstellung relativ zueinander um einen Drehpunkt (122) rotierbar aneinander gelagert sind,
und/oder
b) ein Kugelgelenk (100) umfasst.

6. Medizinische Führungsvorrichtung nach einem der voranstehenden Ansprüche, **dadurch gekennzeichnet, dass** die Halteeinrichtung (14) einen Halteeinrichtungsgrundkörper (54) aufweist und dass das erste Koppelelement (90) am Halteeinrichtungsgrundkörper (54) gehalten ist.

7. Medizinische Führungsvorrichtung nach Anspruch 6, **dadurch gekennzeichnet, dass** zwischen dem ersten Koppelelement (90) und dem Halteeinrichtungsgrundkörper (54) eine Einschnürung (126) oder ein Verbindungssteg (128) ausgebildet ist und dass die Einschnürung (126) oder der Verbindungssteg (128) in der Justier- und in der Koppelstellung einen Koppelspalt (120) der Gelenkkugelaufnahme (98) durchsetzt.

8. Medizinische Führungsvorrichtung nach einem der voranstehenden Ansprüche, **gekennzeichnet durch** eine Feststelleinrichtung (144) zum kraft- und/oder formschlüssigen Halten der Halteeinrichtung (14) und der Sägeschablone (16) in der Sägestellung.

9. Medizinische Führungsvorrichtung nach Anspruch 8, **dadurch gekennzeichnet, dass** die Feststelleinrichtung (144)
a) in Form einer Rast-, Schnapp- und/oder Klemmeinrichtung (146) ausgebildet ist
und/oder
b) mindestens ein erstes Feststellelement (148) umfasst, welches in der Sägestellung direkt oder indirekt das erste Koppelelement (90) gegen das zweite Koppelelement (94) drückt.

10. Medizinische Führungsvorrichtung nach Anspruch 9, **dadurch gekennzeichnet, dass**
a) die Feststelleinrichtung (144) mindestens ein zweites Feststellelement (150) umfasst und dass das mindestens eine erste Feststellelement (148) das mindestens eine zweite Feststellelement (150) in der Sägestellung gegen das erste Koppelelement (90) und dieses gegen das zweite Koppelelement (94) drückt
und/oder
b) das mindestens eine erste Feststellelement (148) einen um eine Exzenterschwenkachse (172) verschwenkbaren Exzenterkörper (168) umfasst.

11. Medizinische Führungsvorrichtung nach Anspruch 10, **dadurch gekennzeichnet, dass** das mindestens eine zweite Feststellelement (150)
a) ein an der Sägeschablone (16) angeordnetes oder ausgebildetes Blattfederelement (156) umfasst
und/oder
b) einen Andrückkörper (178) umfasst
und/oder
c) beweglich an der Sägeschablone (16) angeordnet oder ausgebildet ist
und/oder
d) in einem Spalt (118) oder einer Aussparung zwischen den mindestens zwei hohlzylindrischen Wandabschnitten (106) gehalten ist.

12. Medizinische Führungsvorrichtung nach Anspruch 10 oder 11, **dadurch gekennzeichnet, dass**
a) die Exzenterachse (172) quer, insbesondere senkrecht, zur Koppelelementlängsachse (104) verläuft
und/oder
b) die Exzenterachse (172) parallel oder im Wesentlichen parallel zum Führungsschlitz (18) verläuft
und/oder
c) das mindestens eine erste Feststellelement (148) ein Betätigungsglied (174) umfasst,
welches Betätigungsglied (174) insbesondere vom Exzenterkörper (168) abstehend angeordnet oder ausgebildet ist.

13. Medizinische Führungsvorrichtung nach einem der voranstehenden Ansprüche, **dadurch gekennzeichnet, dass** an der Halteeinrichtung (14) und/oder an der Sägeschablone (16) mindestens eine Befestigungselementaufnahme (64, 130) für eine Knochenbefestigungseinrichtung (66, 138) angeordnet oder ausgebildet ist.

14. Medizinische Führungsvorrichtung nach einem der voranstehenden Ansprüche, **gekennzeichnet durch** eine Positioniereinrichtung (12), welche mit der Halteeinrichtung (14) in einer Positionierstellung gekoppelt ist.

15. Medizinische Führungsvorrichtung nach Anspruch 14, **dadurch gekennzeichnet, dass** die Positioniereinrichtung (12) mindestens ein Knochenanlageelement mit einer Knochenanlagefläche und mindestens ein erstes Kupplungselement (32) einer Kupplungseinrichtung (34) umfasst, welches mindestens eine erste Kupplungselement (32) in der Positionierstellung mit mindestens einem zweiten Kupplungselement (36) der Kupplungseinrichtung (34), welches an der Halteeinrichtung (14) angeordnet oder ausgebildet ist, kraft- und/oder formschlüssig in Eingriff steht.

## Claims

1. Medical guide device (10) for working on a bone, this guide device (10) including a holding arrangement (14), which is holdable firmly against a bone, for holding the guide device (10) firmly against the bone and a saw template (16) with a guide slot (18) for a saw blade, wherein, in an adjustment position, the holding arrangement (14) and the saw template (16) engage with one another and are arranged to be movable in relation to one another, wherein the holding arrangement (14) and the saw template (16; 16') are movable from the adjustment position into a sawing position in which they are held firmly and immovably in relation to one another, this guide device (10) including a coupling arrangement (92) for coupling the holding arrangement (14) and the saw template (16; 16') in the adjustment position and in the sawing position, wherein the coupling arrangement (92) includes first and second coupling elements (90, 94; 90, 94') which are arranged or constructed on the one hand on the holding arrangement (14) and on the other on the saw template (16; 16'), are in engagement with one another in the adjustment position, and are held against one another with force locking and/or positive locking in the sawing position, wherein the first coupling element is constructed in the form of a joint ball (96), wherein the second coupling element is constructed in the form of a joint ball receptacle (98), wherein the joint ball receptacle (96) defines a hollow cylindrical or substantially hollow cylindrical internal contour (102) and makes it possible not only to rotate a joint ball (96) held therein about a centre point defined by the joint ball but also to displace the joint ball (96) relative to the joint ball receptacle (98) parallel to a longitudinal axis (104) defined by the joint ball receptacle (98), **characterised in that** the coupling arrangement (92) makes it possible once the saw template (16) is oriented as desired to transfer the first and second coupling elements (90, 94; 90, 94') from the adjustment position to the sawing position in order to hold the saw template (16) in the latter in the defined orientation.

2. Medical guide device according to Claim 1, **characterised in that** there is arranged or constructed on the holding arrangement (14) and/or on the saw template (16) an interface (70) for a medical referencing arrangement (72) whereof the position and/or orientation in space is detectable by a medical navigation system (86), and/or **in that** a medical referencing arrangement (72) whereof the position and/or orientation in space is detectable by a medical navigation system (86) is arranged or constructed on the holding arrangement (14) and/or the saw template (16).

3. Medical guide device according to Claim 2, **characterised in that**
a) the interface (70) is constructed in the form of a first connecting element (76) which is constructed to correspond to a second connecting element (78) of the medical referencing arrangement (72), and **in that** the first and the second connecting element (76, 78) are in engagement in a connected position and are disengaged in a cleaning position
and/or
b) the medical referencing arrangement (72) bears at least one marker element whereof the position in space is detectable by a medical navigation system (86).

4. Medical guide device according to any one of the preceding claims, **characterised in that**
a) the coupling arrangement (92) is constructed such that, in the adjustment position, the saw template (16) and the holding arrangement (14) are mounted on one another such that they are displaceable in relation to one another
and/or
b) the second coupling element (94) defines a coupling element longitudinal axis (104), and **in that**, in the adjustment position, the saw template (16) and the holding arrangement (14) are mounted on one another such that they are displaceable in relation to one another parallel to the coupling element longitudinal axis (104)
and/or
c) the coupling arrangement (92) is constructed such that, in the adjustment position, the saw template (16) and the holding arrangement (14) are mounted on one another such that they are pivotal in relation to one another about a pivot axis (124).

5. A medical guide device according to any one of the preceding claims, **characterised in that**
a) the coupling arrangement (92) is constructed such that, in the adjustment position, the saw template (16) and the holding arrangement (14) are mounted on one another such that they are rotatable in relation to one another about an axis of rotation (122)
and/or
b) the coupling arrangement (92) includes a ball joint (100).

6. A medical guide device according to any one of the preceding claims, **characterised in that** the holding arrangement (14) has a holding arrangement base body (54), and **in that** the first coupling element (90) is held on the holding arrangement base body (54).

7. A medical guide device according to Claim 6, **characterised in that** a narrowed portion (126) or a connecting web (128) is constructed between the first coupling element (90) and the holding arrangement base body (54), and **in that**, in the adjustment position and the coupling position, the narrowed portion (126) or connecting web (128) passes through a coupling gap (120) in the joint ball receptacle (98).

8. Medical guide device according to any one of the preceding claims, **characterised by** a fixing arrangement (144) for holding the holding arrangement (14) and the saw template (16) in the sawing position with force locking and/or positive locking.

9. Medical guide device according to Claim 8, **characterised in that** the fixing arrangement (144)
a) is constructed in the form of a latching, snap-fitting and/or clamping arrangement (146)
and/or
b) includes at least one first fixing element (148) which, in the sawing position, presses the first coupling element (90) directly or indirectly against the second coupling element (94).

10. Medical guide device according to Claim 9, **characterised in that**
a) the fixing arrangement (144) includes at least one second fixing element (150) and **in that**, in the sawing position, the at least one first fixing element (148) presses the at least one second fixing element (150) against the first coupling element (90) and presses the latter against the second coupling element (94)
and/or
b) the at least one first fixing element (148) includes an eccentric body (168) that is pivotal about an eccentric pivot axis (172).

11. Medical guide device according to Claim 10, **characterised in that** the at least one second fixing element (150)
a) includes a leaf spring element (156) that is arranged or constructed on the saw template (16)
and/or
b) includes a contact pressure body (178)
and/or
c) is arranged or constructed to be movable on the saw template (16)
and/or
d) is held in a gap (118) or a recess between the at least two hollow cylindrical wall portions (106).

12. Medical guide device according to Claim 10 or 11, **characterised in that**
a) the eccentric axis (172) runs transversely, in particular perpendicular, to the coupling element longitudinal axis (104)
and/or
b) the eccentric axis (172) runs parallel or substantially parallel to the guide slot (18)
and/or
c) the at least one first fixing element (148) includes an actuating member (174), this actuating member (174) being arranged or constructed in particular to project away from the eccentric body (168).

13. Medical guide device according to any one of the preceding claims, **characterised in that** at least one securing element receptacle (64, 130) for a bone securing arrangement (66, 138) is arranged or constructed on the holding arrangement (14) and/or the saw template (16).

14. Medical guide device according to any one of the preceding claims, **characterised by** a positioning arrangement (12) which is coupled to the holding arrangement (14) in a positioning disposition.

15. Medical guide device according to Claim 14, **characterised in that** the positioning arrangement (12) includes at least one bone abutment element having a bone abutment surface and at least one first coupling element (32) of a coupling arrangement (34), wherein, in the positioning disposition, the at least one first coupling element (32) is in engagement with force and/or positive locking with at least one second coupling element (36) of the coupling arrangement (34) that is arranged or constructed on the holding arrangement (14).

## Revendications

1. Dispositif de guidage médical (10) pour l'usinage d'un os,
ledit dispositif de guidage (10) comprenant un dispositif de maintien (14) pouvant être fixé à l'os et destiné à fixer le dispositif de guidage (10) à l'os, et un gabarit de sciage (16) avec une fente de guidage (18) pour une lame de scie,
le dispositif de maintien (14) et le gabarit de sciage (16) étant, dans une position d'ajustage, en prise réciproque et agencés de manière réglable l'un par rapport à l'autre,
le dispositif de maintien (14) et le gabarit de sciage (16 ; 16") pouvant être amenés de la position d'ajustage à une position de sciage dans laquelle ils sont fixés de manière immobile l'un par rapport à l'autre,
ledit dispositif de guidage (10) comprenant également un système de couplage (92) pour assurer le couplage du dispositif de maintien (14) et du gabarit de sciage (16 ; 16") dans la position d'ajustage et dans la position de sciage,
le système de couplage (92) comprenant des premier et deuxième éléments de couplage (90, 94 ; 90, 94"), qui sont agencés ou réalisés d'une part sur le dispositif de maintien (14) et d'autre part sur le gabarit de sciage (16 ; 16"), et qui, dans la position d'ajustage, sont en prise réciproque et, dans la position de sciage, sont maintenus l'un contre l'autre par adhérence et/ou complémentarité de formes,
le premier élément de couplage (90) étant réalisé sous la forme d'une rotule d'articulation (96),
le deuxième élément de couplage étant réalisé sous la forme d'un logement d'accueil de rotule d'articulation (98),
le logement d'accueil de rotule d'articulation (98) définissant un contour intérieur (102) cylindrique creux ou sensiblement cylindrique creux, et permettant non seulement la rotation de la rotule d'articulation (96), qui y est maintenue, autour d'un centre défini par la rotule d'articulation (96), mais également un coulissement de la rotule d'articulation (96) par rapport au logement d'accueil de rotule d'articulation (98) parallèlement à un axe longitudinal (104) défini par le logement d'accueil de rotule d'articulation (98),
**caractérisé en ce que** le dispositif de couplage (92) permet, après orientation souhaitée du gabarit de sciage (16), de transférer les premier et deuxième éléments de couplage (90, 94 ; 90, 94') de la position d'ajustage à la position de sciage, pour, dans cette position de sciage, maintenir le gabarit de sciage (16) dans son orientation définie.

2. Dispositif de guidage médical selon la revendication 1, **caractérisé en ce que** sur le dispositif de maintien (14) et/ou sur le gabarit de sciage (16) est agencée ou formée une interface (70) pour un système de relevé de référence médical (72), dont la position et l'orientation dans l'espace peuvent être détectées par un système de navigation médical (86), et/ou **en ce que** sur le dispositif de maintien (14) et/ou sur le gabarit de sciage (16) est agencé ou réalisé un système de relevé de référence médical (72), dont la position et l'orientation dans l'espace peuvent être détectées par un système de navigation médical (86).

3. Dispositif de guidage médical selon la revendication 2, **caractérisé en ce que**
a) l'interface (70) est réalisée sous la forme d'un premier élément de liaison (76), qui est réalisé de manière à correspondre à un deuxième élément de liaison (78) du système de relevé de référence médical (72), et **en ce que** le premier et le deuxième élément de liaison (76, 78) sont en prise réciproque dans une position de liaison, et sont hors de prise dans une position de nettoyage,
et/ou
b) le système de relevé de référence médical (72) porte au moins un élément marqueur dont la position dans l'espace peut être détectée par un système de navigation médical (86).

4. Dispositif de guidage médical selon l'une des revendications précédentes, **caractérisé en ce que**
a) le dispositif de couplage (92) est d'une configuration telle, que le gabarit de sciage (16) et le dispositif de maintien (14) soient montés l'un sur l'autre de manière à pouvoir coulisser l'un par rapport à l'autre dans la position d'ajustage,
et/ou
b) le deuxième élément de couplage (94) définit un axe longitudinal d'élément de couplage (104), et **en ce que** le gabarit de sciage (16) et le dispositif de maintien (14) sont montés l'un sur l'autre de manière à pouvoir coulisser l'un par rapport à l'autre, parallèlement à l'axe longitudinal d'élément de couplage (104), dans la position d'ajustage,
et/ou
c) le dispositif de couplage (92) est d'une configuration telle, que le gabarit de sciage (16) et le dispositif de maintien (14) soient montés l'un sur l'autre de manière à pouvoir pivoter l'un par rapport à l'autre autour d'un axe de pivotement (124), dans la position d'ajustage.

5. Dispositif de guidage médical selon l'une des revendications précédentes, **caractérisé en ce que** le dispositif de couplage (92)
a) est d'une configuration telle, que le gabarit de sciage (16) et le dispositif de maintien (14) soient montés l'un sur l'autre de manière à pouvoir tourner l'un par rapport à l'autre autour d'un centre de rotation (122), dans la position d'ajustage,
et/ou
b) comprend une articulation à rotule (100).

6. Dispositif de guidage médical selon l'une des revendications précédentes, **caractérisé en ce que** le dispositif de maintien (14) comprend un corps de base de dispositif de maintien (54), et **en ce que** le premier élément de couplage (90) est maintenu sur le corps de base de dispositif de maintien (54).

7. Dispositif de guidage médical selon la revendication 6, **caractérisé en ce qu'**entre le premier élément de couplage (90) et le corps de base de dispositif de maintien (54) est formé un rétrécissement (126) ou une branche de liaison (128), et **en ce que** le rétrécissement (126) ou la branche de liaison (128) traverse, dans la position d'ajustage et dans la position de couplage, un interstice de couplage (120) du logement d'accueil de rotule d'articulation (98).

8. Dispositif de guidage médical selon l'une des revendications précédentes, **caractérisé par** un dispositif d'immobilisation (144) pour assurer un maintien par adhérence et/ou complémentarité de formes du dispositif de maintien (14) et du gabarit de sciage (16), dans la position de sciage.

9. Dispositif de guidage médical selon la revendication 8, **caractérisé en ce que** le dispositif d'immobilisation (144)
a) est réalisé sous la forme d'un dispositif d'encliquetage, d'enclenchement et/ou de serrage (146),
et/ou
b) comprend au moins un premier élément d'immobilisation (148), qui, dans la position de sciage, presse directement ou indirectement le premier élément de couplage (90) contre le deuxième élément de couplage (94).

10. Dispositif de guidage médical selon la revendication 9, **caractérisé en ce que**
a) le dispositif d'immobilisation (144) comprend au moins un deuxième élément d'immobilisation (150), et **en ce que** ledit au moins un premier élément d'immobilisation (148) presse, dans la position de sciage, ledit au moins un deuxième élément d'immobilisation (150) contre le premier élément de couplage (90) et celui-ci contre le deuxième élément de couplage (94),
et/ou
b) que ledit au moins un premier élément d'immobilisation (148) comprend un corps d'excentrique (168) pouvant pivoter autour d'un axe de pivotement d'excentrique (172).

11. Dispositif de guidage médical selon la revendication 10, **caractérisé en ce que** ledit au moins un deuxième élément d'immobilisation (150)
a) comprend un élément de ressort à lame (156) agencé ou formé sur le gabarit de sciage (16),
et/ou
b) comprend un corps de pressage (178),
et/ou
c) est agencé ou formé de manière mobile sur le gabarit de sciage (16),
et/ou
d) est maintenu dans un interstice (118) ou une encoche entre au moins deux tronçons de paroi cylindrique creuse (106).

12. Dispositif de guidage médical selon la revendication 10 ou la revendication 11, **caractérisé en ce que**
a) l'axe d'excentrique (172) s'étend transversalement, de préférence de manière perpendiculaire, à l'axe longitudinal d'élément de couplage (104),
et/ou
b) l'axe d'excentrique (172) s'étend parallèlement ou sensiblement de manière parallèle à la fente de guidage (18),
et/ou
c) ledit au moins un premier élément d'immobilisation (148) comprend un organe d'actionnement (174), cet organe d'actionnement (174) étant agencé ou formé notamment de manière à faire saillie du corps d'excentrique (168).

13. Dispositif de guidage médical selon l'une des revendications précédentes, **caractérisé en ce que** sur le dispositif de maintien (14) et/ou sur le gabarit de sciage (16) est agencé ou formé au moins un logement d'accueil d'élément de fixation (64, 130) pour un dispositif de fixation à l'os (66, 138).

14. Dispositif de guidage médical selon l'une des revendications précédentes, **caractérisé par** un dispositif de positionnement (12), qui est couplé au dispositif de maintien (14) dans une situation de positionnement.

15. Dispositif de guidage médical selon la revendication 14, **caractérisé en ce que** le dispositif de positionnement (12) comprend au moins un élément d'appui sur l'os avec une surface d'appui sur l'os, et au moins un premier élément d'accouplement (32) d'un dispositif d'accouplement (34), ledit au moins un premier élément d'accouplement (32) étant, dans la situation de positionnement, en prise par adhérence et/ou par complémentarité de formes avec au moins un deuxième élément d'accouplement (36) du dispositif d'accouplement (34), qui est agencé ou formé sur le dispositif de maintien (14).
